(19)

Europäisches
Patentamt
European
Patent Office
Office européen
des brevets

(11)     **EP 3 754 030 A1**

(12)     **EUROPEAN PATENT APPLICATION**

(43) Date of publication:
**23.12.2020 Bulletin 2020/52**

(51) Int Cl.:
***C12Q 1/6886*** *(2018.01)*

(21) Application number: **19382527.0**

(22) Date of filing: **21.06.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Fundació Institut Mar d'Investigacions
Mèdiques
(IMIM)
08003 Barcelona (ES)**

(72) Inventor: **Bellmunt Molins, Joaquim
08003 BARCELONA (ES)**

(74) Representative: **ZBM Patents - Zea, Barlocci &
Markvardsen
Rambla Catalunya, 123
08008 Barcelona (ES)**

(54)     **GENOMIC PREDICTOR OF OUTCOME IN HIGH GRADE T1 NON-MUSCLE INVASIVE BLADDER
CANCER**

(57)     The present invention refers to an *in vitro* method for the prediction of the outcome in a subject suffering High grade T1 (HGT1) non-muscle invasive bladder cancer (NMIBC), the method comprising the step of identifying at least a somatic mutation in the ERCC2 gene and/or identifying the COSMIC 2 signature and/or the COSMIC5 signatures in an isolated sample from the subject. The present invention also refers to an *in vitro* method for deciding or recommending a medical regime in to a subject with HGT1 NMIBC.

EP 3 754 030 A1

**Description**

**Technical Field**

The present invention relates to the field of Medicine, particularly to bladder cancer, specifically to a new method for predicting the outcome of High grade T1 (HGT1) non-muscle-invasive bladder cancer. The method has potential applications in the clinical management of HGT1 cancer patients, in terms of medical treatment, such as radical cystectomy recommendation.

**Background Art**

There are approximately 430,000 new cases of bladder cancer worldwide annually, with 165,000 associated cancer deaths. When initially diagnosed, approximately 75% of patients have non-muscle-invasive bladder cancer (NMIBC), either confined to the mucosa (Ta and carcinoma *in situ* [CIS]) or invading the bladder lamina propria but not the muscularis propria (T1).

The subgroup of high-grade T1 (HGT1) bladder cancer is a high-risk subtype of NMIBC, with highly variable prognosis and poorly understood risk factors. HGT1 tumors can present with similar histopathological characteristics but behaving clinically with different disease aggressiveness. The risk of recurrence is estimated to be about 40% while the risk of progression to muscle-invasive bladder cancer (MIBC) is approximately seen in 21% of cases at 5 years. Nowadays, no validated predictive method exists for an adequate management of HGT1 tumors.

In the era of cancer genomics, several attempts have been made to find drivers of progression and recurrence in HGT1 tumors. A meta-analysis, identified selected prognostic clinical pathological variables (Martin-Doyle W, *et al.,* 2015), but without sufficient power in clinical decision making. Expression signatures using microarrays have been evaluated, with a lack of confirmation across different studies (Dyrskjøt L, *et al.,* 2017). Mutational signatures, mutational signatures clustering or mutation and copy number clustering have not been explored as tools to discriminate between variable disease outcomes to identify which HGT1 NMIBC patients are going to develop recurrence or progression or conversely will never recur or progress.

Actually, the decision to offer conservative local treatment with transurethral resection of bladder tumor (TURBT), intravesical bacillus Calmette-Guerin (BCG) and close surveillance, or radical cystectomy (RC), is currently made in the absence of validated prognostic biomarkers. RC entails surgery on the urinary tract, intestines, and lymph nodes; hence, complications frequently occur after this invasive procedure. It is well established the use of RC in the case of doubt, but there are times when the RC does not work, for example, when implemented too late, not preventing subsequent progression or metastasis. Less aggressive treatments are available, such as TURBT and/or intravesical BCG but the decision of their recommendation is done with limited guidance, without the help of a helpful predictive tool.

Despite the efforts made, there is a need of tools which allow accurate prediction of the progression of a patient suffering HGT1 NMIBC.

**Summary of Invention**

The inventors have found some valuable informative markers about the prediction of the outcome of HGT1 NMIBC.

In particular, the inventors have found that the identification of at least a somatic mutation in the ERCC2 gene in an isolated sample of a subject with High grade T1 (HGT1) non-muscle invasive bladder cancer (NMIBC) can provide useful information about the prognosis of HGT1 NMIBC in said subject.

In addition, the inventors have also found that COSMIC 2 signature is able to provide useful information; whereas COSMIC 5 signature, when combined with at least a somatic mutation in the ERCC2 gene, is able to provide useful information about the prognosis of HGT1 NMIBC in said subject.

By performing whole exome sequencing of HGT1 NMIBC it was found that overall mutational burden was increased in good outcome (GO) compared to progression (PD), with recurrent (R) patients having the lowest mutational burden (p=0.017). DNA-damage response (DDR) gene mutations was associated with GO (p=0.009) and with higher mutational burden (p<0.0001). Enrichment analysis of significantly mutated genes in each outcome group showed that ERCC2 was significantly associated to GO (q=0.1, empirical p-value (p#)=0.003 by random-permutation and p*=0.022 by one-tailed Fisher's exact) (see section 3 of the results). The most common somatic mutations of ERCC2 were missense mutations (see table 1).

By performing *de novo* mutational signature analysis, the inventors have identified two signatures associated with outcome (see section 5 of the results): the activity of the COSMIC5 signature was significantly higher in ERCC2 mutant samples and was also associated with GO (p=0.0002); and the COSMIC 2 signature was highly prevalent in this cohort of HGT1 (30%) and was associated with GO (p=0.047). ERCC2 mutation was associated with overall Somatic Single Nucleotide Variant (SNVs) indicating that the increase of mutation burdens in GO was partly attributed to the activity of COSMIC5 in addition to the APOBEC mutagenesis.

From the data provided below, it is remarkable the fact that when there were identified somatic mutations of the ERCC2 gene, the COSMIC 2 and/or COSMIC 5 signatures in a sample, then the patient with HGT1 NMIBC was associated with good outcome (see section 9 of the results).

Thus, the first aspect of the invention refers to an *in vitro* method for the prediction of the outcome in a subject suffering High grade T1 (HGT1) non-muscle invasive bladder cancer (NMIBC), the method comprising the step of identifying at least a somatic mutation in the ERCC2 gene and/or identifying the COSMIC 2 signature in an isolated sample from the subject.

The results provided below support that the presence or absence of somatic mutations of the ERCC2 gene, the COSMIC 2 and/or COSMIC 5 signatures in an urothelial carcinoma of a patient are informative of prognosis of HGT1 NMIBC; being the presence indicative of good outcome, whereas the absence is indicative of bad outcome. These prognosis markers can help in the decision or recommendation of a medical regime of HGT1 NMIBC based on radical cystectomy or on a less risky medical regime, such TURBT and/or intravesical BCG. Therefore, the invention means a great advance in accurately predicting the outcome of a patient already diagnosed from HGT1 NMIBC. This can be of great value for the physician in order to decide the best therapeutic strategy to successfully overcome the disease, such as to undergo radical cystectomy or to avoid it.

Therefore, a second aspect of the invention refers to an *in vitro* method for deciding or recommending a medical regime to a subject with HGT1 NMIBC, the method comprising the step of identifying at least one somatic mutation of the ERCC2 gene and/or the COSMIC 2 signature; or, alternatively, at least a somatic mutation of the ERCC2 gene and the COSMIC 5 signature; or, alternatively, at least a somatic mutation of the ERCC2 and at least one DNA-damage response gene somatic mutation, wherein when it is a check-point gene it is selected from ATR, CHEK1, CHEK2 or MDC1; or, alternatively, at least a somatic mutation of the ERCC2 and at least one DNA-damage response gene somatic mutation wherein it is a nucleotide excision repair gene somatic mutation; or, alternatively, at least a somatic mutation of the ERCC2, ERCC3, ERCC4 and ERCC5 genes; or, alternatively, at least a somatic mutation of ERCC2 and BRCA2 genes; or, alternatively, the COSMIC 2 signature and the COSMIC 5 signature; or, alternatively, the COSMIC 2 signature, at least one somatic mutation of the ERCC2 gene and the COSMIC 5 signature, or, alternatively, at least a somatic mutation in ERCC2, CREBBP, KMT2D, KMT2C, EP300 and in the RB gene; or, alternatively, at least one somatic mutation in ERCC2, KMT2C and the COSMIC 5 signature are identified; in an isolated biological sample of the patient; and when the somatic mutation(s) is(are) identified, the medical regime decided or recommended is transurethral resection of bladder tumor (TURBT) and/or intravesical bacillus Calmette-Guerin (BCG).

A third aspect of the invention refers to the use of somatic mutations of the ERCC2 gene and/or of the COSMIC 2 signature, as marker(s) of prediction of outcome of HGT1 NMIBC or of deciding or recommending a medical regimen to a patient with HGT1 NMIBC.

A fourth aspect of the invention refers to the use of means for identifying the somatic mutations and/or quantifying the gene copy number of the genes as defined in any one of the aspects of the invention, in any of the *in vitro* methods as defined in any of the first or second aspects of the present invention

**Brief Description of Drawings**

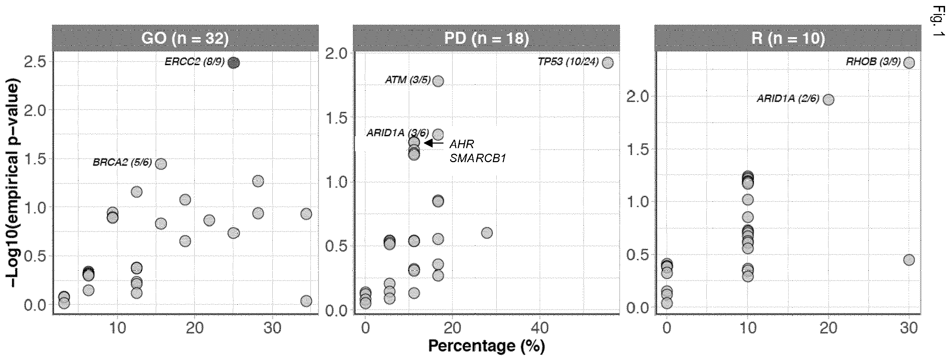

Fig. 1 Outcome association of bladder cancer-related genes (mutated in >=4 samples). The percentage of samples with non-silent mutations is shown in the x axis and the empirical p-value (p#) from the random-permutation method, partially correcting heterogeneous mutation burdens among different outcome groups, is shown on the y axis. The named genes refers to p value <0.05. The false-discovery rate (FDR) value for ERCC2 in GO was < 0.1.

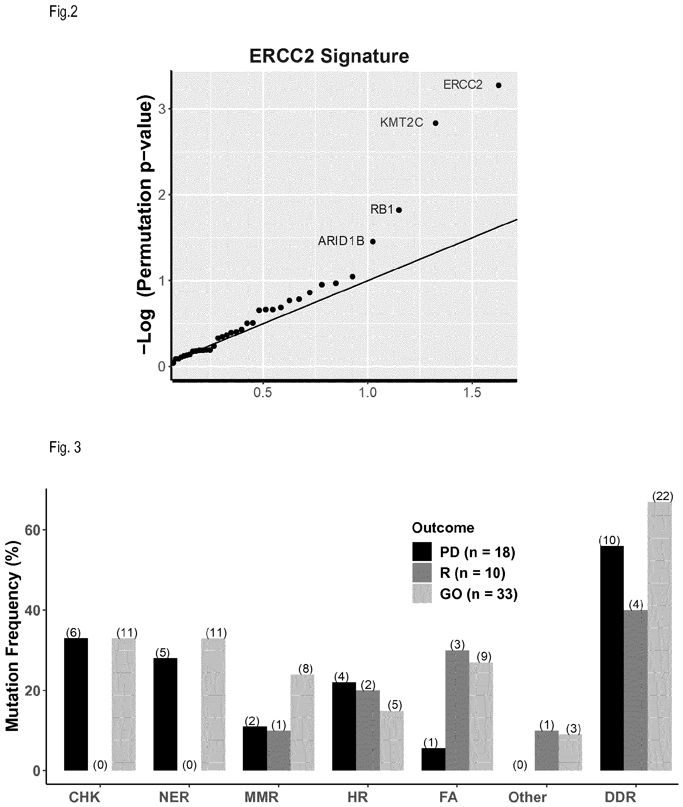

Fig. 2 Enrichment of the mutations in ERCC2 (COSMIC5) signatures. Q-Q plots showing observed versus expected p-values for each of the analyses. For genes ERCC2 and KMT2C q value (qval) <0.2 and for the genes RB1 and ARID1B p value (p-val) <0.05. As was done in the outcome association analysis, it was only considered genes seen in $\geq$ 3 patients (5% of cohort).

Fig. 3 shows the number of patients (n=61, POLE sample excluded) with mutations in DDR genes (subdivided into smaller gene sets) grouped by disease outcome. DDR: DNA damage repair, MMR: Mismatch repair, NER: Nucleotide excision repair, HR: Homologous recombination, FA: Fanconi anemia.

**Detailed description of the invention**

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition. The definitions given herein are included for the purpose of understanding and expected to be applied throughout description, claims and drawings.

The term "outcome" means prognosis. The term "prognosis" is the natural evolution of the disease in terms of survival if there is no treatment in between.

The term "good outcome" (GO) refers to no recurrence of the bladder cancer after at least 4 years of follow-up in a subject previously identified with HGT1 NMIBC. Good outcome is therefore, good prognosis.

The term "bad outcome" comprises recurrence and progression.

The term "recurrent disease" refers to the appearance of a bladder cancer with the same stage or less appeared before 4 years in the same subject previously identified with HGT1 NMIBC.

The term "progressive disease" (PD) refers to the appearance of progression to muscle-invasion or metastasis during the follow-up period (for example, 4 years) in the same subject previously identified with HGT1 NMIBC.

The ERCC2 gene is the ERCC excision repair 2, TFIIH core complex helicase subunit; the gene identification number in the public database GenBank® is Gene ID: 2068 (on 21 May 2019).

In the present invention the genes cited herein are described below with their full name and their number identifier in the public data base GenBank® (see the material and method section and table 2).

"Somatic mutations" are mutations not from the germ line. Somatic mutations are present in all cells of the human body. They occur throughout life. They are the consequence of multiple mutational processes, for example, by the DNA replication machinery, exogenous or endogenous mutagen exposures, enzymatic modification of DNA and defective DNA repair. Different mutational processes generate unique combinations of mutation types, termed "Mutational Signatures".

The term "signatures" refers to different combinations of mutation types generated by different mutational processes.

The "COSMIC 2 signature" (also known as "COSMIC signature 2", "COSMIC 2", "COSMIC2", "APOBEC 2 signature", and "APOBEC-a signature") is well known by the expert in the field of somatic mutations in cancer (COSMIC is the acronym of "Catalogue of Somatic Mutations in Cancer") (Alexandrov LB *et al.* 2013). COSMIC 2 signature is characterized by predominant C>T transitions and some C>G transversions at TC[A/T] motifs (where the mutated C is preceded by T and followed by A or T), (C->T at TCW, (W=A/T)).

"COSMIC 5 signature" (or "Cosmic signature 5", "signature 5" or "COSMIC 5" or "COSMIC5") is well known by the expert in the field of somatic mutations in cancer. COSMIC 5 signature has been found in all cancer types and most cancer samples. Signature 5 exhibits transcriptional strand bias for T>C substitutions at ApTpN context, as described for example in Alexandrov LB *et al.* 2013.

In the present invention, when a signature is identified, this means that all the mutations of said signature are detected.

The first aspect of the invention refers to an *in vitro* method for the prediction of the outcome in a subject suffering High grade T1 (HGT1) non-muscle invasive bladder cancer (NMIBC), the method comprising the step of identifying at least a somatic mutation in the ERCC2 gene and/or identifying the COSMIC 2 signature in an isolated sample from the subject

In an embodiment of the first aspect of the present invention, optionally in combination with any of the embodiments provided below, the method further comprises the step of identifying the COSMIC 5 signature.

In a further embodiment of the first aspect of the present invention, optionally in combination with any of the embodiments provided above or below, it is identified at least a somatic mutation in the ERCC2 gene and the COSMIC 5 signature; or, alternatively, at least a somatic mutation in the ERCC2 gene, the COSMIC 2 signature and the COSMIC 5 signature are identified.

In a further embodiment of the first aspect of the present invention, optionally in combination with any of the embodiments provided above or below, when at least one somatic mutation it is identified in the ERCC2 gene; or, alternatively, the COSMIC 2 signature is identified, it is indicative of good outcome.

In a further embodiment of the first aspect of the present invention, optionally in combination with any of the embodiments provided above or below, when at least a somatic mutation of the ERCC2 gene and the COSMIC signature 5 are identified, or, alternatively, wherein when the COSMIC 2 signature and the COSMIC signature 5 are identified; or, alternatively, wherein when at least one somatic mutation of the ERCC2 gene, the COSMIC 2 signature, and the COSMIC signature 5 are identified, it is indicative of good outcome.

In the present invention somatic mutations and copy number alterations of certain genes have been associated with the prediction of outcome when they are combined with the presence or absence of somatic mutations in the ERCC2 gene, the COSMIC 2 signature and/or the COSMIC 5 signature.

ERCC2 belongs to DNA damage response (DDR) genes, in particular to the nuclear excision repair (NER) genes. When focusing on NER genes, it was found that mutations in the NER genes to be predictors of good outcome (see section 4 of the results). The NER genes analysed and associated with GO were ERCC2, ERCC3, ERCC4 and ERCC5 (see table 2).

Also, BRCA2 (p value<0.05) was found as a main driver of GO as it was enriched in said samples (see section 3 of the results).

The identification of copy-number clusters allowed to identify that good outcome was also predicted identifying at least a somatic mutation in ERCC2, CREBBP, KMT2D, KMT2C, EP300 and in the RB1 gene (see section 8 of the results). Good prognosis was also associated with somatic mutations in ERCC2, in KMT2C and the COSMIC5 signature (see for example, fig. 2).

When somatic mutations in the ERCC2 gene, the COSMIC 2 signature and/or COSMIC 5 signature were not detected but at least one somatic TP53 mutation, CCNE1 copy number gain and/or mutations in CDKN2A were detected, this was indicative of bad outcome with disease progression (see section 9 of the results).

TP53 mutations and focal somatic copy number alterations in the CCNE1, PVRL4, YWHAZ, E2F3, SOX4 and PPARG genes were associated with a cluster associated with bad outcome (see section 8 of the results).

Thus, in another embodiment of the first aspect of the present invention, optionally in combination with any of the embodiments provided above or below, the method further comprises one or more of the following steps, in any order:

a. Identifying at least one somatic mutation in one or more genes selected from the following list: a DNA-damage response gene, RB1, CREBBP, KMT2D, KMT2C, EP300, TP53, ATM, ARID1A, AHR, SMARCB1, RHOB, FGFR3 and KDM6A;
b. Determining the copy number of one or more genes selected from the following list: RB1, CCNE1, CDKN2A, PVRL4, YWHAZ, E2F3, SOX4 and PPARG;
c. Evaluating at least one or more clinical-pathologic factor selected form the following list: lamina propria invasion, lymphovascular invasion, associated carcinoma *in situ,* use of bacillus Calmette-Guerin (BCG) and tumor size.

Thus, in a further embodiment of the first aspect of the present invention, optionally in combination with any of the embodiments provided above or below, the method further comprises one or more of the following steps, in any order:

a. Identifying at least one somatic mutation in one or more genes selected from the following list: a DNA-damage response gene, RB1, CREBBP, KMT2D, KMT2C, EP300 and TP53;
b. Determining the copy number of one or more genes selected from the following list: RB1, CCNE1, CDKN2A, PVRL4, YWHAZ, E2F3, SOX4 and PPARG;
c. Evaluating at least one or more clinical-pathologic factor selected form the following list: lamina propria invasion, lymphovascular invasion, associated carcinoma *in situ,* use of bacillus Calmette-Guerin (BCG) and tumor size.

In a further embodiment of the first aspect of the present invention, optionally in combination with any of the embodiments provided above or below, the somatic mutations identified is ERCC2, RB1, CREBBP, KMT2D, KMT2C and EP300 mutations.

In a further embodiment of the first aspect of the present invention, optionally in combination with any of the embodiments provided above or below, the somatic mutation further identified is at least one TP53 mutation.

In a further embodiment of the first aspect of the present invention, optionally in combination with any of the embodiments provided above or below, the somatic mutation further identified is a TP53 mutation, CCNE1 copy number gain and/or CDKN2A deletion. In further embodiment of the first aspect of the present invention, optionally in combination with any of the embodiments provided above or below, the somatic mutation further identified is a TP53 mutation, CCNE1 copy number gain and CDKN2A deletion.

In a further embodiment of the first aspect of the present invention, optionally in combination with any of the embodiments provided above or below, it is further identified TP53, ATM, ARID1A, AHR and SMARCB1 somatic mutations.

In a further embodiment of the first aspect of the present invention, optionally in combination with any of the embodiments provided above or below, it is further identified at least one ARID1A somatic mutation.

In a further embodiment of the first aspect of the present invention, optionally in combination with any of the embodiments provided above or below, it is further identified at least one FGFR3, KDM6A and/or KMT2D somatic mutation.

In a further embodiment of the first aspect of the present invention, optionally in combination with any of the embodiments provided above or below, it is further identified at least one RHOB somatic mutation.

In a further embodiment of the first aspect of the present invention, optionally in combination with any of the embodiments provided above or below, it is further identified at least one somatic mutation of RHOB and ARID1A.

In a further embodiment of the first aspect of the present invention, optionally in combination with any of the embodiments provided above or below, the somatic mutation further identified is a TP53 mutation and somatic copy number alterations (SCNAs) in CCNE1, PVRL4, YWHAZ, E2F3, SOX4 and PPARG.

In a further embodiment of the first aspect of the present invention, optionally in combination with any of the embodiments provided above or below, the *in vitro* method comprises the identification of one or more of the DNA-damage response gene(s).

In a further embodiment of the first aspect of the present invention, optionally in combination with any of the embodiments provided above or below, it is identified one or more of the DNA-damage response gene(s) wherein it is a nucleotide excision repair gene, mismatch repair gene, a homologous recombination gene, a Fanconi anemia gene, or a check-point gene, wherein the check-point gene is ATR, CHEK1, CHEK2 or MDC1.

In a further embodiment of the first aspect of the present invention, optionally in combination with any of the embodiments provided above or below, the DNA-damage response genes are genes selected form the same group or from a different group of the ones indicated above.

In a further embodiment of the first aspect of the present invention, optionally in combination with any of the embodiments provided above or below, the nucleotide excision repair gene is a gene selected form the following list: ERCC3, ERCC4 or ERCC5.

In a further embodiment of the first aspect of the present invention, optionally in combination with any of the embodiments provided above or below, the mismatch repair gene is a gene selected form the following list: MLH1, MSH2, MSH6, PMS1 or PMS2.

In a further embodiment of the first aspect of the present invention, optionally in combination with any of the embodiments provided above or below, the homologous recombination gene is a gene selected form the following list: BRCA1, MRE11A, NBN, RAD50, RAD51, RAD51B, RAD51D, RAD52 or RAD54L.

In a further embodiment of the first aspect of the present invention, optionally in combination with any of the embodiments provided above or below, the Fanconi anemia gene is a gene selected form the following list: BRCA2, BRIP1, FANCA, FANCC, PALB2, RAD51C or BLM.

In a further embodiment of the first aspect of the present invention, optionally in combination with any of the embodiments provided above or below, the check-point gene is a gene selected form the following list: ATM, ATR, CHEK1, CHEK2 or MDC1.

In a further embodiment of the first aspect of the present invention, optionally in combination with any of the embodiments provided above or below, the DNA-damage response gene is selected from the following list: MLH1, MSH2, MSH6, PMS1, PMS2, ERCC3, ERCC4, ERCC5, BRCA1, MRE11A, NBN, RAD50, RAD51, RAD51B, RAD51D, RAD52, RAD54L, BRCA2, BRIP1, FANCA, FANCC, PALB2, RAD51C, BLM, ATM, ATR, CHEK1, CHEK2, MDC1, POLE, MUTYH, PARP1 or RECQL4.

In a further embodiment of the first aspect of the present invention, optionally in combination with any of the embodiments provided above or below, the *in vitro* method comprises identifying at least a somatic mutation in ERCC2 gene and of at least one of a DNA-damage response gene.

In a further embodiment of the first aspect of the present invention, optionally in combination with any of the embodiments provided above or below, the *in vitro* method comprises identifying at least a somatic mutation in ERCC2 gene and of at least one of a DNA-damage response gene which is a nucleotide excision repair gene.

In a further embodiment of the first aspect of the present invention, optionally in combination with any of the embodiments provided above or below, the *in vitro* method for the prediction of the outcome comprises the steps of identifying at least a somatic mutation in ERCC2 and of at least one or more nucleotide excision repair gene(s) selected form the following list: ERCC3, ERCC4, ERCC5; or, alternatively, the steps of identifying at least a somatic mutation in ERCC2, ERCC3, ERCC4 and ERCC5.

A further embodiment of the first aspect of the present invention, optionally in combination with any of the embodiments provided above or below, refers to the method wherein the DNA-damage response gene is BRCA2. In another embodiment, optionally in combination with any of the embodiments provided above or below, the method comprises the steps of identifying at least a somatic mutation in ERCC2 gene as well as in BRCA2.

In a further embodiment of the first aspect of the present invention, optionally in combination with any of the embodiments provided above or below, when it is identified at least a somatic mutation of the ERCC2 and at least one DNA-damage response gene somatic mutation are identified, wherein when it is a check-point gene it is selected from ATR, CHEK1, CHEK2 or MDC1; or, alternatively, at least a somatic mutation of the ERCC2 and at least another one DNA-damage response gene wherein it is a nucleotide excision repair gene somatic mutation are identified; or, alternatively, at least a somatic mutation of the ERCC2, ERCC3, ERCC4 and ERCC5 genes are identified; or, alternatively, at least a somatic mutation of ERCC2 and BRCA2 genes are identified; or, alternatively, at least one somatic mutation in ERCC2, CREBBP, KMT2D, KMT2C, EP300 and in the RB1 gene, or, alternatively, at least one somatic mutation in ERCC2, KMT2C and COSMIC 5 signature are identified, this is indicative of good outcome.

In a further embodiment of the first aspect of the present invention, optionally in combination with any of the embodiments provided above or below, when it is further identified at least one FGFR3 and/or KDM6A somatic mutation, then this is indicative of good outcome.

A further embodiment of the first aspect of the present invention, optionally in combination with any of the embodiments provided above or below, refers to the method wherein when somatic mutations of the ERCC2 gene and/or the COSMIC 2 signature is not identified; or, alternatively, wherein when somatic mutations of the ERCC2 gene and the COSMIC 5 signature are not identified; or, alternatively, wherein somatic mutations of the ERCC2 gene and another one DNA-damage response gene somatic mutations are not identified, wherein when it is a check-point gene it is selected from ATR, CHEK1, CHEK2 or MDC1; or, alternatively, wherein somatic mutations of the ERCC2 gene and another one DNA-damage response gene wherein it is a nucleotide excision repair gene somatic mutation are not identified; or, alternatively, wherein somatic mutations of the ERCC2, ERCC3, ERCC4 and ERCC5 genes are not identified; or, alternatively, wherein somatic mutations of ERCC2 and BRCA2 genes are not identified; or, alternatively, wherein when the COSMIC 2 signature and the COSMIC 5 signature are not identified; or, alternatively, wherein when the COSMIC 2 signature, or at least one somatic mutation of the ERCC2 gene and the COSMIC 5 signature are not identified; or, alternatively, wherein at least one somatic mutation in ERCC2, CREBBP, KMT2D, KMT2C, EP300 and in the RB1 gene are not identified; or, alternatively, at least one somatic mutation in ERCC2, KMT2C and COSMIC 5 signature are not identified, this is indicative of bad outcome of the HGT1 NMIBC.

A further embodiment of the first aspect of the present invention, optionally in combination with any of the embodiments provided above or below, refers to the method wherein when somatic mutations of the ERCC2 gene and/or the COSMIC 2 signature is not identified; or, alternatively, wherein when somatic mutations of the ERCC2 gene and the COSMIC 5 signature are not identified; or, alternatively, wherein somatic mutations of the ERCC2 gene and another one DNA-damage response gene somatic mutations are not identified, wherein when it is a check-point gene it is selected from ATR, CHEK1, CHEK2 or MDC1; or, alternatively, wherein somatic mutations of the ERCC2 gene and another one DNA-damage response gene wherein it is a nucleotide excision repair gene somatic mutation are not identified; or, alternatively, wherein somatic mutations of the ERCC2, ERCC3, ERCC4 and ERCC5 genes are not identified; or, alternatively, wherein somatic mutations of ERCC2 and BRCA2 genes are not identified; or, alternatively, wherein when the COSMIC 2 signature and the COSMIC 5 signature are not identified; or, alternatively, wherein when the COSMIC 2 signature, at least one somatic mutation of the ERCC2 gene and the COSMIC 5 signature are not identified, or, alternatively, wherein at least one somatic mutation in ERCC2, CREBBP, KMT2D, KMT2C, EP300 and in the RB1 gene are not identified; or, alternatively, at least one somatic mutation in ERCC2, KMT2C and COSMIC 5 signature are not identified; and wherein at least one somatic mutation in TP53, copy number gain of CCNE1 and/or CDKN2A deletion are identified, or, alternatively, when at least one somatic mutation in TP53 gene and somatic copy number alterations (SCNAs) in CCNE1, PVRL4, YWHAZ, E2F3, SOX4 and PPARG are identified; and/or, when is identified lamina propria invasion, lymphovascular invasion, associated carcinoma *in situ*, non-use of bacillus Calmette-Guerin (BCG), and/or tumor size >3 cm, then this is indicative of the bad outcome of HGT1 NMIBC wherein the bad outcome is the progression of the HGT1 NMIBC.

A further embodiment of the first aspect of the present invention, optionally in combination with any of the embodiments provided above or below, refers to the method wherein when at least one somatic mutation of the TP53 genes is identified and there are further identified somatic mutations of the ATM, ARID1A, AHR and SMARCB1 genes; then, this is indicative of the bad outcome of HGT1 NMIBC wherein the bad outcome is the progression of the HGT1 NMIBC.

A further embodiment of the first aspect of the present invention, optionally in combination with any of the embodiments provided above or below, refers to the method wherein when somatic mutations of the ERCC2 gene and/or the APOBEC2 signature is not identified; or, alternatively, wherein when somatic mutations of the ERCC2 gene and the COSMIC 5 signature are not identified; or, alternatively, wherein somatic mutations of the ERCC2 gene and another one DNA-damage response gene somatic mutations are not identified, wherein when it is a check-point gene it is selected from ATR, CHEK1, CHEK2 or MDC1; or, alternatively, wherein somatic mutations of the ERCC2 gene and another one DNA-damage response gene wherein it is a nucleotide excision repair gene somatic mutation are not identified; or, alternatively, wherein somatic mutations of the ERCC2, ERCC3, ERCC4 and ERCC5 genes are not identified; or, alternatively, wherein somatic mutations of ERCC2 and BRCA2 genes are not identified; or, alternatively, wherein when the COSMIC 2 signature and the COSMIC 5 signature are not identified; or, alternatively, wherein when the COSMIC 2 signature, at least one somatic mutation of the ERCC2 gene and the COSMIC 5 signature are not identified, or, alternatively, wherein at least one somatic mutation in ERCC2, CREBBP, KMT2D, KMT2C, EP300 and in the RB1 gene are not identified; or, alternatively, at least one somatic mutation in ERCC2, KMT2C and COSMIC 5 signature are not identified; and wherein at least one somatic mutation in TP53 gene and somatic copy number alterations (SCNAs) in CCNE1, PVRL4, YWHAZ, E2F3, SOX4 and PPARG are identified, it is indicative of recurrence or progression.

A further embodiment of the first aspect of the present invention, optionally in combination with any of the embodiments provided above or below, refers to the method wherein when somatic mutations of the ERCC2 gene and/or the APOBEC2 signature is not identified; or, alternatively, wherein when somatic mutations of the ERCC2 gene and the COSMIC 5 signature are not identified; or, alternatively, wherein somatic mutations of the ERCC2 gene and another one DNA-damage response gene somatic mutations are not identified, wherein when it is a check-point gene it is selected from ATR, CHEK1, CHEK2 or MDC1; or, alternatively, wherein somatic mutations of the ERCC2 gene and another one DNA-damage response gene wherein it is a nucleotide excision repair gene somatic mutation are not identified; or, alternatively, wherein somatic mutations of the ERCC2, ERCC3, ERCC4 and ERCC5 genes are not identified; or, alternatively, wherein somatic mutations of ERCC2 and BRCA2 genes are not identified; or, alternatively, wherein when the COSMIC 2 signature and the COSMIC 5 signature are not identified; or, alternatively, wherein when the COSMIC 2 signature, at least one somatic mutation of the ERCC2 gene and the COSMIC 5 signature are not identified, or, alternatively, wherein at least one somatic mutation in ERCC2, CREBBP, KMT2D, KMT2C, EP300 and in the RB1 gene are not identified; or, alternatively, at least one somatic mutation in ERCC2, KMT2C and COSMIC 5 signature are not identified; and wherein at least one somatic mutation in RHOB and/or ARID1A, is (are) identified, it is indicative of recurrence.

The second aspect of the invention refers to an *in vitro* method for deciding or recommending a medical regime in to a subject with HGT1 NMIBC, the method comprising the steps of identifying at least one somatic mutation of the ERCC2 gene and/or the COSMIC 2 signature; or, alternatively, at least a somatic mutation of the ERCC2 gene and the COSMIC 5 signature; or, alternatively, at least a somatic mutation of the ERCC2 and at least one DNA-damage response gene somatic mutation, wherein when it is a check-point gene it is selected from ATR, CHEK1, CHEK2 or MDC1; or, alternatively, at least a somatic mutation of the ERCC2 and at least one DNA-damage response gene somatic mutation wherein it is a nucleotide excision repair gene somatic mutation; or, alternatively, at least a somatic mutation of the ERCC2, ERCC3, ERCC4 and ERCC5 genes; or, alternatively, at least a somatic mutation of ERCC2 and BRCA2 genes; or, alternatively, the COSMIC 2 signature and the COSMIC 5 signature; or, alternatively, the COSMIC 2 signature , at least one somatic mutation of the ERCC2 gene and the COSMIC 5 signature, or, alternatively, at least a somatic mutation in ERCC2, CREBBP, KMT2D, KMT2C, EP300 and in the RB1 gene; or, alternatively, at least one somatic mutation in ERCC2, KMT2C and COSMIC 5 signature are identified; in an isolated biological sample of the patient; and when the somatic mutation(s) is(are) identified, the medical regime decided or recommended is transurethral resection of bladder tumor (TURBT) and/or intravesical bacillus Calmette-Guerin (BCG). Radical cystectomy is not recommended in this case.

The term "radical cystectomy" is the surgery to remove the entire urinary bladder.

The embodiments of the first aspect of the invention are also embodiments of the second aspect of the invention.

In a further embodiment of the second aspect of the present invention, optionally in combination with any of the embodiments provided above or below, it is identified one or more of the DNA-damage response gene(s).

In a further embodiment of the second aspect of the present invention, optionally in combination with any of the embodiments provided above or below, the DNA-damage response gene(s) it is a nucleotide excision repair gene, mismatch repair gene, a homologous recombination gene, a Fanconi anemia gene, or a check-point gene, wherein the check-point gene is ATR, CHEK1, CHEK2 or MDC1.

In a further embodiment of the second aspect of the present invention, optionally in combination with any of the embodiments provided above or below, the DNA-damage response genes are genes selected form the same group or from a different group of the ones indicated above.

In a further embodiment of the second aspect of the present invention, optionally in combination with any of the embodiments provided above or below, the nucleotide excision repair gene is a gene selected form the following list: ERCC3, ERCC4 or ERCC5.

In a further embodiment of the second aspect of the present invention, optionally in combination with any of the embodiments provided above or below, the mismatch repair gene is a gene selected form the following list: MLH1, MSH2, MSH6, PMS1 or PMS2.

In a further embodiment of the second aspect of the present invention, optionally in combination with any of the embodiments provided above or below, the homologous recombination gene is a gene selected form the following list: BRCA1, MRE11A, NBN, RAD50, RAD51, RAD51B, RAD51D, RAD52 or RAD54L.

In a further embodiment of the second aspect of the present invention, optionally in combination with any of the embodiments provided above or below, the Fanconi anemia gene is a gene selected form the following list: BRCA2, BRIP1, FANCA, FANCC, PALB2, RAD51C or BLM.

In a further embodiment of the second aspect of the present invention, optionally in combination with any of the embodiments provided above or below, the check-point gene is a gene selected form the following list: ATR, CHEK1, CHEK2 or MDC1.

In a further embodiment of the second aspect of the present invention, optionally in combination with any of the embodiments provided above or below, the DNA-damage response gene is selected from the following list: MLH1, MSH2, MSH6, PMS1, PMS2, ERCC3, ERCC4, ERCC5, BRCA1, MRE11A, NBN, RAD50, RAD51, RAD51B, RAD51D, RAD52, RAD54L, BRCA2, BRIP1, FANCA, FANCC, PALB2, RAD51C, BLM, ATM, ATR, CHEK1, CHEK2, MDC1, POLE, MUTYH, PARP1 or RECQL4.

In a further embodiment of the second aspect of the present invention, optionally in combination with any of the embodiments provided above or below, it comprises identifying at least a somatic mutation in ERCC2 gene and of at least one of a DNA-damage response gene.

In a further embodiment of the second aspect of the present invention, optionally in combination with any of the embodiments provided above or below, it comprises identifying at least a somatic mutation in ERCC2 gene and of at least one of a DNA-damage response gene which is a nucleotide excision repair gene.

In a further embodiment of the second aspect of the present invention, optionally in combination with any of the embodiments provided above or below, it comprises, the steps of identifying at least a somatic mutation in ERCC2 and of at least one or more nucleotide excision repair gene(s) selected form the following list: ERCC3, ERCC4, ERCC5; or, alternatively, the steps of identifying at least a somatic mutation in ERCC2, ERCC3, ERCC4 and ERCC5.

In a further embodiment of the second aspect of the present invention, optionally in combination with any of the embodiments provided above or below, it is further identified at least one FGFR3 and/or KDM6A somatic mutation, and wherein it is identified, then the medical regime decided or recommended is transurethral resection of bladder tumor (TURBT) and/or intravesical bacillus Calmette-Guerin (BCG). Radical cystectomy is not recommended in this case.

In a further embodiment of the second aspect of the present invention, optionally in combination with any of the embodiments provided above or below, the *in vitro* method for deciding or recommending a medical regime further comprises one or more of the following steps in any order:

- identifying at least one somatic mutation of one or more gene selected from the following list: TP53, ATM, ARID1A, AHR, SMARCB1 and RHOB;
- determining the copy number of one or more genes selected from the following list: CCNE1, CDKN2A, PVRL4, YWHAZ, E2F3, SOX4 and PPARG;
- evaluating one or more clinical-pathologic factor(s) selected form the following list: lamina propria invasion, lymphovascular invasion, associated carcinoma *in situ,* use of bacillus Calmette-Guerin (BCG), and tumor size;

wherein, when no somatic mutation(s) as defined in the second aspect of the invention is detected, but at least one somatic mutation is detected in TP53, copy number gain of CCNE1 and/or CDKN2A deletion are identified; or, alternatively, when at least one somatic mutation in TP53 gene is identified; or, alternatively, when at least one somatic mutation in TP53, ATM, ARID1A, AHR, and/or SMARCB1 genes are identified; or, alternatively, when at least one somatic mutation in RHOB1 and/or ARID1A gene is(are) identified; or, alternatively, when at least one somatic mutation in TP53 gene and SCNAs in CCNE1, PVRL4, YWHAZ, E2F3, SOX4 and PPARG are identified; or, alternatively, when one or more of lamina propria invasion, lymphovascular invasion, associated carcinoma *in situ,* non-use of bacillus Calmette-Guerin (BCG) and/or tumor size >3 cm is detected, the medical regime decided or recommended is radical cystectomy.

In a further embodiment of the second aspect of the present invention, optionally in combination with any of the embodiments provided above or below, the treatment decided or recommended is radical cystectomy; in an example, the BCG is administered before the radical cystectomy.

In a further embodiment of the first or the second aspect of the present invention, optionally in combination with any of the embodiments provided above or below, the somatic mutation is a somatic mutation in an exon.

In a further embodiment of the first or the second aspect of the present invention, optionally in combination with any of the embodiments provided above or below, the somatic mutation of ERCC2 is a mutation in the coding region of the ERCC2 gene.

In a further embodiment of the first or the second aspect of the present invention, optionally in combination with any of the embodiments provided above or below, the somatic mutation of ERCC2 is a missense mutation.

In a further embodiment of the first or the second aspect of the present invention, optionally in combination with any of the embodiments provided above or below, the somatic mutation identification is performed by an amplifying or sequencing of the nucleic acid comprised in the isolated sample.

In a further embodiment of the first or the second aspect of the present invention, optionally in combination with any of the embodiments provided above or below, the amplification technique is a PCR based technique.

In a further embodiment of the first or the second aspect of the present invention, optionally in combination with any of the embodiments provided above or below, the mutation is identified in the DNA or in the mRNA comprised in the isolated sample.

In one embodiment of any of the methods provided by the present the invention, optionally in combination with any of the embodiments provided above or below, it is identified the mutations for each one of the gene markers provided by the invention via polymerase chain reaction (using, for example, oligonucleotide primers). In particular embodiments of the invention, optionally in combination with any of the embodiments provided above or below, RT-PCR can be used to amplify the mRNA for protein cancer markers for detection and analysis.

Alternatively, in another embodiment of any of the methods provided by the present invention, optionally in combination with any of the embodiments provided above or below, it is determined the amount of mRNA for each one of the gene markers provided by the invention via a hybridization technique, employing oligonucleotide probes.

When using mRNA, the method may be carried out by combining isolated mRNA with reagents to convert to cDNA according to standard methods well known in the art, treating the converted cDNA with amplification reaction reagents (such as cDNA PCR reaction reagents) in a container along with an appropriate mixture of nucleic acid primers; reacting the contents of the container to produce amplification products; and analyzing the amplification products to detect the presence of one or more of the cancer markers in the sample. The analysis step may be further accomplished by quantitatively detecting the presence of polynucleotide cancer markers in the amplification product and comparing the quantity of marker detected against a panel of expected values for the known presence or absence of such markers in normal and malignant tissue derived using similar primers.

In a further embodiment of the first or the second aspect of the present invention, optionally in combination with any of the embodiments provided above or below, the sample is an isolated bladder tissue sample or a biological fluid.

In a further embodiment of the first or the second aspect of the present invention, optionally in combination with any of the embodiments provided above or below, the biological fluid is urine, blood or plasma.

In a further embodiment of the first or the second aspect of the present invention, optionally in combination with any of the embodiments provided above or below, the sample is fresh, frozen, fixed or fixed and embedded in paraffin; in an example, the sample is a paraffin embedded bladder cancer tissue.

In the present invention the terms "patient", "subject" and "individual" are used interchangeably.

In the present invention the patient is a mammal, preferably is a human. The patient can be of any age, gender or race.

The third aspect of the present invention refers to the use of somatic mutations of the ERCC2 gene and/or the COSMIC 2 signature as marker(s) of prediction of outcome of HGT1 NMIBC or of deciding or recommending a medical regimen to a patient with HGT1 NMIBC.

A further embodiment of the third aspect of the present invention, optionally in combination with any of the embodiments provided above or below, refers to the combined use of at least one somatic mutation of the ERCC2 gene and the COSMIC 5 signature as markers of prediction of outcome of HGT1 NMIBC or of deciding or recommending a medical regimen to a patient with HGT1 NMIBC.

A further embodiment of the third aspect of the present invention, optionally in combination with any of the embodiments provided above or below, refers to the combined use of at least one of the somatic mutations of ERCC2 and BRCA2 genes; or, alternatively, of the combined use of at least one of the somatic mutations of the ERCC2 gene and of another one DNA-damage response gene; or, alternatively, of the combined use of at least one of the somatic mutations of the ERCC2 gene and of another one DNA-damage response gene wherein it is a nucleotide excision repair gene; or, alternatively, of the combined use of at least one of the somatic mutations of the ERCC2, ERCC3, ERCC4 and ERCC5 genes; or, alternatively, of the combined use of the APOBEC2 signature and the COSMIC 5 signature; or, alternatively, of the combined use of the COSMIC 2 signature, at least one somatic mutation of the ERCC2 gene and the COSMIC 5 signature; or, alternatively, of the combined use of at least one of the somatic mutations of the ERCC2, CREBBP, KMT2D, KMT2C, EP300 and the RB1 genes; or, alternatively, at least one somatic mutation in ERCC2, KMT2C and COSMIC 5 signature, as marker(s) of prediction of outcome of HGT1 NMIBC or of deciding or recommending a medical regimen to a patient with HGT1 NMIBC.

The fourth aspect of the present invention refers to the use of means for identifying the somatic mutations as defined in any one of the aspects of the invention, in any of the *in vitro* methods as defined in any of the *in vitro* methods of the invention.

In a further embodiment of the fourth aspect of the present invention, optionally in combination with any of the embodiments provided above or below, the means form part of a kit.

The nature of the means depends on the technique selected to identify the gene or the signature. Details about their nature have been provided above (primers, probes, antibodies, and fluorescent dyes, among others). The kit may additionally comprise further means (additives, solvents) to visualize the interactions (dipsticks, chemiluminescent reagents, turbidimetric reagents, etc.). Suitable additives, solvents and reagents to visualize the identification are disclosed in the examples.

The *in vitro* methods of the invention provide prognostic information. In one embodiment, the methods of the invention further comprise the steps of (i) collecting the prognostic information, and (ii) saving the information in a data carrier.

In the sense of the invention a "data carrier" is to be understood as any means that contain meaningful information data for the prognosis of HGT1 NMIBC, such as paper. The carrier may also be any entity or device capable of carrying the prognosis data. For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the prognosis data are embodied in a signal that may be conveyed directly by a cable or other device or means, the carrier may be constituted by such cable or other device or means. Other carriers relate to USB devices and computer archives. Examples of suitable data carrier are paper, CDs, USB, computer archives in PCs, or sound registration with the same information.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

## Examples

### 1. Example 1: prognostic factors for HGT1 NMIBC:

### Material and methods:

### Demographic, Clinical, and Pathological Data:

Seventy seven clinically annotated samples of high grade T1 (HGT1) urothelial carcinoma were obtained from two different centers in Spain (Hospital del Mar, and University Hospital Vall d'Hebron [HVH], both in Barcelona, Spain). Whole exome sequencing (WES) was performed on these archival, formalin-fixed, paraffin-embedded tissue. Only 62 out of 77 tumor-only (TO) samples harbored a sufficient coverage for the tumor-only mutation-calling pipeline.

The study cohort begun on April 2005 with last assessment performed in February 2014 with a median follow-up time of 7.4 years (range from 2 to 9 years). All patients were treated with BCG following initial diagnosis. Follow-up included cystoscopy plus cytology alternated with TUR every three months for the first two years and every six months until five years thereafter. Clinical outcome was classified as good outcome (GO) when no recurrence was seen after at least 4 years of follow-up, recurrent disease (R) when recurrent disease with the same stage or less appeared before 4 years or progressive disease (PD) when progression to muscle-invasion or metastasis was seen during this follow-up period. The Dana Farber Cancer Institute, Hospital del Mar and HVH Ethics Committee granted study approval. At last assessment, the outcome was GO in 33 patients, R in 10 patients, PD in 18 and 1 with unknown clinical data.

In an attempt to identify predictors of clinical outcome in this subgroup of patients, a metanalysis of published HGT1 studies was performed to address the question of which clinical-pathologic prognostic factors were predictors of subsequent recurrence, progression and cancer specific survival (Martin-Doyle W *et al.* 2015).

### Patient samples and clinical information

Three pathologists reviewed and selected the cases. An independent review and selection of the tumor-rich regions and normal areas for sequencing was done by a fourth pathologist (JB). Study approval was granted by the Dana Farber Cancer Institute, Hospital del Mar and HVH Ethics Committee. Patients with concomitant CIS were excluded. Tumors were graded according to the 2004 WHO system (2006) after pathological assessment of the total specimen.

### Nucleic acid isolation

From a series of 128 formalin-fixed paraffin embedded (FFPE) tumor samples, 10micron (mu) cores were obtained from FFPE tumor blocks from areas previous identified by an expert genitourinary pathologist. DNA was extracted from FFPE tumor areas and "normal" bladder areas using the QIAamp DNA Mini Kit (Qiagen). DNA was extracted from normal bladder areas using a Nucleon DNA extraction kit (Nucleon Biosciences) or by salt precipitation.

To characterize the mutational landscape of HGT1 NMIBC whole exome sequencing (WES) was performed on 62 evaluable samples without a paired normal sample (tumor-only, TO) and 15 for which there was a corresponding germline sample (matched tumor/normal, T/N). Somatic single nucleotide variant mutations (SNVs) and indels were called by standard methods. Mutation burden, mutation signature, copy number alterations (CNA) and mutational signature clustering analysis alone or combined with CNA were also analyzed, blinded to clinical status.

### DNA sequencing and data processing

WES was performed at the Broad Institute. All methods were standard at the Broad Institute, and identical to those used for The Cancer Genome Atlas (TCGA) projects (Robertson AG et al. 2017 Cell 171(3):540-556).

Briefly, exome capture was performed using the Agilent SureSelect Human All Exon 50 Mb probe set, with 0.2 to 0.5 micrograms of DNA, and sequencing libraries were prepared by standard methods including addition of indexed sequencing adaptors. FFPE DNA gave results comparable to high-quality DNA. 77 HGT1 tumor and 38 matched normal samples had sufficient amount of DNA extracted for WES pipeline. Preliminary QC analysis found tumor-in-normal contamination in -50% of matched normal samples. Due to limitations on good quality DNA yield, only 62 TO samples harbored a sufficient coverage for the tumor-only mutation-calling pipeline; 15 of these samples had corresponding germ line tissues qualified for the final analysis. The "field-disease" mutations in cells with almost normal phenotype in the light microscope when coring "normal" urothelial close to the tumor, can explain this high degree of contamination. The Picard and Firehose pipelines (described in Strasky N. *et al.* 2011) were used to perform basic alignment of reads using Burrows-Wheeler Aligner (Li H, *et al.* 2009) on the University California Santa Cruz (UCSC) human reference genome version hg19, quality control to make sure tumor and normal are matched; local realignment of reads at site of indels in the reads; identification of somatic SNVs in the tumor compared to normal (or panel of normals) using the Mutect (Cibulskis K *et al.* 2013) algorithm (version 1); and identification of somatic insertions and deletions using the Indelocator algorithm. The final output from this analysis was a Mutation Annotation Format or maf file that listed all variants detected and additional information, including: the read frequency of the variant allele, the read frequency of the reference allele, category of mutation, genomic coordinates, cDNA coordinates, protein coordinates, and information on whether the gene was found to be mutated in the COSMIC database.

All variants in the maf file using IGV 2.4 were reviewed (Robinson JT, *et al.* 2011). Variants were removed if there were artifacts due to repetitive sequence or misalignments, known SNPs, had minor allele frequency <5%, were present in intergenic regions or had no effect on coding sequence, or cause a synonymous amino acid change.

Genomic findings were correlated to clinical outcome, differentiating between GO, R and PD urothelial HGT1 carcinomas. In addition, it was explored the possible association between DNA-based clusters (van der Heijden AG, *et al.* 2016) and the pathological microstaging.

**Tumor only analysis**

**a. Gene selection**

96 genes were selected for downstream analyses based on significantly mutated genes (SMG) found in the 412 TCGA samples (Robertson AG et al. 2017 Cell 171 (3):540-556) adding manually curated panel of genes relevant to NMIBC described in the literature. The genes were the following (in brackets the name and the Gene ID number in the GenBank data base on the 21st May 2019): AHR (aryl hydrocarbon receptor, Gene ID: 196), AKT1 (AKT serine/threonine kinase 1, Gene ID: 207), APC (APC regulator of WNT signaling pathway, Gene ID: 324), ARID1A (AT-rich interaction domain 1A, Gene ID: 8289), ARID1B (AT-rich interaction domain 1B, Gene ID: 57492), ARID2 (AT-rich interaction domain 2, Gene ID: 196528), ASXL2 (ASXL transcriptional regulator 2, Gene ID: 55252), ATM (ATM serine/threonine kinase, Gene ID: 472), BRCA1 (BRCA1 DNA repair associated, Gene ID: 672), BRCA2 (BRCA2 DNA repair associated, Gene ID: 675), BRINP1 (BMP/retinoic acid inducible neural specific 1, Gene ID: 1620), BRWD1 (bromodomain and WD repeat domain containing 1, Gene ID: 54014), BTG2 (BTG anti-proliferation factor 2, Gene ID: 7832), CCND1 (cyclin D1, Gene ID: 595), CCND3 (cyclin D3, Gene ID: 896), CCNE1 (cyclin E1, Gene ID: 898), CDH3 (cadherin 3, Gene ID: 1001), CDKN1A (cyclin dependent kinase inhibitor 1A, Gene ID: 1026), CDKN1 B (cyclin dependent kinase inhibitor 1B, Gene ID: 1027), CDKN2A (cyclin dependent kinase inhibitor 2A, Gene ID: 1029), CDKN2B (cyclin dependent kinase inhibitor 2B, Gene ID: 1030), CREBBP (CREB binding protein, Gene ID: 1387), CTNNB1 (catenin beta 1, Gene ID: 1499), DBC1 (BMP/retinoic acid inducible neural specific 1, Gene ID: 1620), E2F1 (E2F transcription factor 1, Gene ID: 1869), E2F3 (E2F transcription factor 3, Gene ID: 1871), EZH2 (enhancer of zeste 2 polycomb repressive complex 2 subunit, Gene ID: 2146), EGFR (epidermal growth factor receptor, Gene ID: 1956), ELF3 (E74 like ETS transcription factor 3, Gene ID: 1999), EP300 (E1A binding protein p300, Gene ID: 2033), ERBB2 (erb-b2 receptor tyrosine kinase 2, Gene ID: 2064), ERBB3 (erb-b2 receptor tyrosine kinase 3, Gene ID: 2065), ERBB4 (erb-b2 receptor tyrosine kinase 4, Gene ID: 2066), ERCC2 (ERCC excision repair 2, TFIIH core complex helicase subunit, Gene ID: 2068), FAM47C (family with sequence similarity 47 member C, Gene ID: 442444), FBXW7 (F-box and WD repeat domain containing 7, Gene ID: 55294), FGFR1 (fibroblast growth factor receptor 1, Gene ID: 2260), FGFR2 (fibroblast growth factor receptor 2, Gene ID: 2263), FGFR3 (fibroblast growth factor receptor 3, Gene ID: 2261), FOXA1 (forkhead box A1, Gene ID: 3169), FOXM1 (forkhead box M1, Gene ID: 2305), FOXQ1 (forkhead box Q1, Gene ID: 94234), GATA3 (GATA binding protein 3, Gene ID: 2625), HORMAD1 (HORMA domain containing 1, Gene ID: 84072), HOXA1 (homeobox A1, Gene ID: 3198), HOXA2 (homeobox A2, Gene ID: 3199), HOXB1 (homeobox B1, Gene ID: 3211), HOXB2 (homeobox B2, Gene ID: 3212), HRAS (HRas proto-oncogene, GTPase, Gene ID: 3265), IDH1 (isocitrate dehydrogenase (NADP(+)) 1 cytosolic, Gene ID: 3417), IRS4 (insulin receptor substrate 4, Gene ID: 8471), KDM6A (lysine demethylase 6A, Gene ID: 7403), KLF12 (Kruppel like factor 12, Gene ID: 11278), KLF5 (Kruppel like factor 5, Gene ID: 688), KMT2A (lysine methyltransferase 2A, Gene ID: 4297), KMT2C (lysine methyltransferase 2C, Gene ID: 58508), KMT2D (lysine methyltransferase 2D, Gene ID: 8085), KRAS (KRAS proto-oncogene, GTPase, Gene ID: 3845), MCL1 (MCL1 apoptosis regulator, BCL2 family member, Gene ID: 4170), MDM2 (MDM2 proto-oncogene, Gene ID: 4193), MITF (melanocyte inducing transcription factor, Gene ID: 4286), MTOR (mechanistic target of rapamycin kinase, Gene ID: 2475), MYBL2 (MYB proto-oncogene like 2, Gene ID: 4605), MYC (MYC proto-oncogene, bHLH transcription factor, Gene ID: 4609), NFE2L2 (nuclear factor, erythroid 2 like 2, Gene ID: 4780), NOTCH1 (notch receptor 1, Gene ID: 4851), NRAS (NRAS proto-oncogene, GTPase, Gene ID: 4893), PIK3CA (phosphatidylinositol-4,5-bisphosphate 3-kinase catalytic subunit alpha, Gene ID: 5290), PIK3R1 (phosphoinositide-3-kinase regulatory subunit 1, Gene ID: 5295), PLK1 (polo like kinase 1, Gene ID: 5347), PPARG (peroxisome proliferator activated receptor gamma, Gene ID: 5468), PRMT5 (protein arginine methyltransferase 5, Gene ID: 10419), PTCH1 (patched 1, Gene ID: 5727), PTEN (phosphatase and tensin homolog, Gene ID: 5728), PVRL4 (nectin cell adhesion molecule 4, Gene ID: 81607), RB1 (RB transcriptional corepressor 1, Gene ID: 5925), RHOA (ras homolog family member A, Gene ID: 387), RHOB (ras homolog family member B, Gene ID: 388), RUNX3 (RUNX family transcription factor 3, Gene ID: 864), RXRA (retinoid X receptor alpha, Gene ID: 6256), SMARCB1 (SWI/SNF related, matrix associated, actin dependent regulator of chromatin, subfamily b, member 1, Gene ID: 6598), SOX4 (SRY-box 4, Gene ID: 6659), STAG2 (stromal antigen 2, Gene ID: 10735), STAT3 (signal transducer and activator of transcription 3, Gene ID: 6774), SUZ12 (SUZ12 polycomb repressive complex 2 subunit, Gene ID: 23512), TET2 (tet methylcytosine dioxygenase 2, Gene ID: 54790), TGFBR1 (transforming growth factor beta receptor 1, Gene ID: 7046), TP53 (tumor protein p53, Gene ID: 7157), TSC1 (TSC complex subunit 1, Gene ID: 7248), TSC2 (TSC complex subunit 2, Gene ID: 7249), TXNIP (thioredoxin interacting protein, Gene ID: 10628), TYRO3 (TYRO3 protein tyrosine kinase, Gene ID: 7301), YWHAZ (tyrosine 3-monooxygenase/tryptophan 5-monooxygenase activation protein zeta, Gene ID: 7534), ZFP36L1 (ZFP36 ring finger protein like 1, Gene ID: 677) and ZFP36L2 (ZFP36 ring finger protein like 2, Gene ID: 678).

### b. Sample selection

The average coverage for each gene for each sample was calculated using the Broad Institute's internal pipeline Firehose (Robertson AG, et al. 2017 Cell 171(3):540-556). Samples were removed from further analysis if more than 25 genes (-25% of 96 selected genes) contained an average coverage of less than 30x sequencing depth. 62 out of 77 tumor samples met this criteria to be considered for further analysis.

### c. SNVs and Indels Calling

The Mutect algorithm (Giacomelli AO, et al. 2018) was used to identify somatic SNVs in targeted exons. Artifacts introduced by DNA oxidation during sequencing or FFPE storage were removed. All mutations were compared to a panel of normal tissue samples (n=27) and removed if they appeared more than once. All samples were processed on the Broad Institute's Firehose.
Mutational frequency of the selected 96 SMGs was analyzed. For consistency, comparison and overlap of findings were made with MC3 PanCancer (Taylor-Weiner A, et al. 2018), and with TCGA muscle invasive bladder cancer (MIBC) (Hedegaard J, et al. 2016). The comparison did show that the frequency of indels from tumor-only samples was too high compared to TCGA data set. In addition, 68 indels from tumor-only were not overlapping with MC3 or TCGA data. Based on that, it was decided to avoid the potential confounding factor of including indels in the association analysis and to ignore all indel calls. The analysis was done with only SNVs.

### d. Germline event filtering

An additional filtering technique utilizing the COSMIC (version 80) database of somatic mutations in cancer (Forbes SA, et al. 2015) and the ExAC database of 60,000 germline samples (Lek M, et al. 2016) was applied to remove additional germline events (Garofalo A, et al. 2016) Missense, Nonsense, Splice Site, and Frame Shift, and In Frame insertions and deletion mutations were selected for and removed if reported in the ExAC database, unless present in the COSMIC database at least 3 times.

### e. Mutation Significance Analysis

MutSig 2CV (Lawrence MS et al. 2013 Nature 499(7457):214-218) was applied to identify significantly mutated genes (SMGs) from the list of filtered variant calls. MutSig evaluates if genes were significantly mutated across the cohort while accounting for factors such as gene length and background mutation rate.

**f. Copy Number Analysis**

The bam files were also analyzed to identify regions of copy number gain or loss. Genome-wide allele frequency distribution plots of all SNPs were made using R to enable identification of regions of copy-neutral LOH. Exome-wide copy-ratios were inferred using CAPSEG and further interrogated using GISTIC 2.0 (Mermel CH, *et al.* 2011). Relative copy ratios reported by GISTIC 2.0 for these genes were converted to log 2 total copy ratios (Eq. 1):

$$\text{log copy ratio} = \log 2(2 + \text{relative copies})$$

GISTIC analysis combined with manual inspection identified focal amplifications as those with log2(copy ratio) > 1 and focal deletions as those with log2(copy ratio) < -0.7.

**g. Mutation Signature Analysis**

A *de-novo* mutational signature analysis of the 62 samples was performed for 27,477 SNVs stratified into 96 base substitution types using "SignatureAnalyzer". This algorithm applied a Bayesian variant of the non-negative matrix factorization (NMF) algorithm with an exponential prior (BayesNMF) (Meeks JJ, *et al.* 2017; Kim J, *et al.* 2016) to enable a *de novo* signature discovery with an optimal inference for the number of signatures (K∗) best explaining the observed mutation counts matrix (96 x 62 samples). Convergence was reached with K∗= 5, identifying five distinct mutational processes, C > T_CpG, POLE, APOBEC-b, and APOBEC-a and COSMIC5 (ERCC2).

A scaling transformation was performed in order to compute signature representation in each sample. The MSig clustering analysis for 62 samples was performed using a standard hierarchical clustering in R, with a 'euclidian' distance for the signature activity matrix H and a 'ward.D' linkage. The number of MSig clusters (Dyrskjøt L, *et al.* 2012) was chosen by manual inspection.

**h. MutCN: Unsupervised clustering of mutations in SMGs and focal SCNAs**

A binary event matrix, Q(n by m) was built comprising mutations in 20 genes SMGs, focal SCNAs in the 9 genes and 11 arm-level SCNAs events across 49 samples (samples with both SNVs and CN data). There were first computed p-values for a pairwise mutual exclusivity among 80 features and applied a BayesNMF to the log10-transformed p-value matrix (as previously done in Robertson AG et al. Cell 171(3):540-556.e25), identifying three DNA-based "Mutation and Copy Number" (MutCN) clusters.

**Tumor/normal pair analysis**

Twenty-seven samples with matched tissue-normal (T/N) WES were subject to the somatic mutation-calling pipeline at Broad (MuTect version 1 and formalin-fixed paraffin embedded [FFPE] and panel of normal filtrations). Twelve samples failed quality control (QC) from a low coverage (<23Mb) and a significant "tumor contamination in normal" (TiN >50%). To rescue mutations rejected by the presence of alternative reads from TiN, DeTiN was ran (Taylor-Weiner A, *et al.* 2018) for the remaining 15 paired samples, yielding 5,956 SNVs with a median 284 mutation per sample, similar to the 319 mutation per sample seen in the corresponding TO-set.

**a. Comparision of TO and T/N calls**

A comparison of TO mutation calls with corresponding paired mutation calls (PAIRED) was performed for 15 tumor-normal pairs assuming that PAIRED calls were ground-truth. A significant drop was seen in the precision (true positive [TP] rate = mutations seen in both TO and PAIRED) with a decrease of variants or an increase of TiN in PAIRED set in the precision, defined as a ratio of TP to the TP and false-positives (FP: mutations only seen in TO), suggesting that a contamination of tumor components in adjacent normal was a significant confounding factor in somatic mutation calling in PAIRED set. On the other hand, the sensitivity, defined as a ratio of TP to the TP and false-negatives (FN: mutations only seen in PAIRED), was rather insensitive to TiN due to a decrease of FN with increasing TiN in normal. Also, it should be noted that tumor purity was another confounding factor. For nine individuals with a sufficient coverage and a minimal TiN level (<3%), the overall sensitivity was 80% and 83% for all non-silent variants and variants in 96 SMGs, and the overall precision was 73% for all non-silent variants and 90% for variants in 96 SMGs. The overall F-score (2TP/(2TP+FP+FN)) was 77% for all non-silent variants and 86% in 96 SMGs.

**b. Mutation analysis in the matched cohort**

All rejected calls were reviewed by the reason of a possible germline contamination or alternative alelle (alt_allele) in tumors not to miss any important mutations. There were four FGFR3 mutations rejected by a coverage issue (less than 10 supporting reads). These FGFR3 mutations were on hotspots, but were not included.

**Statistical analyses**

Mutual exclusivity and coocurrence analyses were done by Fisher's exact test. P values were adjusted for multiple testing resulting in false-discovery rate (FDR) values.

Univariate analysis for the relationship between genomic variables and the three clinical outcome (GO, R, PD) groups was evaluated using Kruskal-Wallis test, and Fisher's exact test, as appropriate. A multivariate logistic regression analysis was performed to evaluate the prognostic association of clinical and genomic variables with good outcome.

Unsupervised clusters to clinical and molecular covariates was compared by calculating contingency table association p values using R, with a Chi-square or Fisher exact test for categorical data, and a Kruskal-Wallis test for real-valued data.

Outcome and signature enrichment analysis

To eliminate the fact that, in general, genes were more likely to be mutated in samples that have a higher mutation burden (inflation), it was applied a permutation test in which it was controlled both the gene-specific and sample-specific mutation counts when generating random permutations of the observed gene $\times$ sample binary mutation matrix, following an approach described previously (Strona G, *et al.* 2014 Nat Commun 5:4114). For signature enrichment it was used as a test statistic, p, the one-tailed Wilcoxon rank-sum p value comparing the signature activities of mutant and wild-type samples of a given gene. For outcome enrichment it was used the one-tailed Fisher's exact p value across genes for each outcome status. It was calculated this test statistic for the observed data, p*, as well as for every realization of the permuted mutation matrix, pk, where k= 1, 2, ..., 50000 (the total number of permutations). The final empirical p value, p#, assigned to the gene was the fraction of permuted realizations with a test statistic more extreme than the observed test statistic (i.e. ones for which pk< p*). By maintaining the row and column margins of the observed mutation matrix in every random realization, it was corrected for the higher tendency of genes to be mutated in samples with higher mutation burden, as evidenced by the fact that nearly all genes except ERCC2 and other few were on the diagonals of the quantile-quantile plots (see figure 2). Because of statistical power and computational efficiency considerations, they were analyzed only genes with a non-silent mutation in >= 4 samples across the analyzed cohort. It was corrected for multiple-hypothesis testing using the Benjamini-Hochberg procedure (Yoav Benjamini and Yosef Hochberg 1995) and used FDR q<0.1 as the significance threshold, unless otherwise specified.

**Results:**

**1. Clinical-pathologic prognostic factors:**

Deep lamina propria invasion (microstaging pT1a vs pT1b) had the largest negative impact. With less powerful prediction of outcome, lymphovascular invasion, associated carcinoma *in situ,* non-use of bacillus Calmette-Guerin (BCG) and tumor size >3 cm, predicted progression and shorter cancer-specific survival (data not shown).

**2. Tumor mutational burdens (TMB)**

As explained in the Material and Methods section, WES was performed on 77 HGT1 tumor-only (TO) samples, wherein only 62 TO samples harbored a sufficient coverage for the tumor-only mutation-calling pipeline. After stringent filtering for germ line variants with COSMIC and ExAC databases, 20,661 single-nucleotide variants (SNVs) affecting coding sequence (missense, nonsense, splice site) and 6,816 silent or non-synonymous mutations were identified. The median mutation rate including both non-silent and silent across 62 TO samples was 8.6 per Mb (median 303 SNVs per sample). The parallel analysis on fifteen samples with matched tissue-normal (T/N) identified 5,956 SNVs with a median of 284 mutations per sample, similar to the median in TO set.

In the present invention the cohort of HGT1 NMIBC, the median non-silent mutation rate across 62 samples was 6.5 per Megabase (Mb).

Interestingly, a significant difference in overall TMB was found among non-progressors, progressors, and recurrent tumors at a median of 9.6/Mb, 7.3/Mb and 5.7/Mb, respectively (p=0.017 by Kruskal-Wallis).

### 3. Significantly Mutated Genes

MutSig 2CV identified 17 genes significantly mutated in more than 10% of the samples (q<0.1; Table 1). TP53 mutation was seen in forty percent of the samples (25 out of 62) and rarely overlapped with MDM2 amplifications (2 out of 10, p=0.14 by one-tailed Fisher's exact). Notably, alterations in TP53 pathway (TP53 mutation or MDM2 amplification) were exclusive with FGFR3 mutation or amplification (p=0.03 by one-tailed Fisher's exact) or TSC1 mutations or deletions (p=0.013 by one-tailed Fisher's exact).

Alterations in chromatin genes were frequent in the cohort. Seven of the 17 SMGs were chromatin modifying or regulatory genes: histone demethylase (KDM6A 24%), histone methyltransferases (KMT2C 21%, KMT2D 27%), histone acetylases (CREBBP 21%, EP300 16%), a member of the SWI/SNF chromatin remodeling complex (ARID1A 11%), and Polycomb group genes (ASXL2 13%). In addition, 9% of tumors showed deletions in CREBBP.

Cell cycle-related genes were the second most frequently altered set of genes, including: CDKN2A deletion (31%), RB1 mutation (18%), and TP53 mutations (40%). Other frequently altered pathway encompassed genes involved in the DNA-damage response (DDR) and genomic regulation (ERCC2 16%, BRCA2 11%, STAG2 13%, ATM 10%). Finally, several genes of the RAS/RTK/PI3K signaling pathway were found to be frequently mutated (FGFR3 13%, PIK3CA 13 %, ERBB2 15%, ERBB3 16%, RHOB 15%). In addition to 17 significantly mutated genes, were also considered other bladder-cancer related genes in downstream analyses, resulting in total 96 genes, profiled from 54 significantly mutated genes in muscle-invasive bladder cancers (MIBC) (2017 TCGA study (Pietzak EJ, *et al.,* 2017) and 42 genes known to be relevant to NMIBC in the literature (see Material and Methods section "Gene selection").

Table 1: Seventeen significantly mutated genes in more than 10% of the samples across 62 tumors by MutSig2CV (q<0.1).

| gene | No. of mutations by type (missense, splice site, nonsense) | No.mut.pat | mut.freq.% in the overall cohort |
|---|---|---|---|
| TP53 | 20, 4, 1 | 25 | 40,3 |
| KMT2D | 7, 2, 8 | 17 | 27,4 |
| KDM6A | 5, 1, 9 | 15 | 24,2 |
| KMT2C | 11, 0, 2 | 13 | 21 |
| CREBBP | 6, 2, 5 | 13 | 21 |
| RB1 | 3, 4, 4 | 11 | 17,7 |
| ERBB3 | 9, 1, 0 | 10 | 16,1 |
| ERCC2 | 10, 0, 0 | 10 | 16,1 |
| EP300 | 4, 2, 4 | 10 | 16,1 |
| RHOB | 9, 0, 0 | 9 | 14,5 |
| ERBB2 | 9, 0, 0 | 9 | 14,5 |
| STAG2 | 4, 1, 3 | 8 | 12,9 |
| PIK3CA | 8, 0, 0 | 8 | 12,9 |
| FGFR3 | 8, 0, 0 | 8 | 12,9 |
| ASXL2 | 6, 0, 2 | 8 | 12,9 |
| ARID1A | 0, 1, 6 | 7 | 11,3 |
| BRCA2 | 7, 0, 0 | 7 | 11,3 |
| "No.mut.pat,", number of patients with at least one mutation; "mut.freq", mutation frequency. | | | |

Heterogeneous mutation burden across samples was a significant confounding factor in identifying associations of genes or mutations with clinical outcome or other covariates. To mitigate this, an enrichment analysis was performed with a partial correction for overall mutation burden using a permutation-based method that maintains the overall number of non-silent mutations per sample and gene (see Methods) (Meeks JJ *et al.* 2017).There were only considered 30 genes seen in ≥4 samples (a single hyper-mutant phenotype sample was excluded) and adjusted empirical p-values by FDR.

ERCC2 was the only significant gene associated to GO (q=0.1, p#=0.003 by random-permutation and p*=0.022 by one-tailed Fisher's exact) (Fig. 1). Overall, 8 out 9 GO patients with an ERCC2 mutation had a non-silent ERCC2 mutation. The p#-value for GO enrichment was less than 0.05 in BRCA2 but didn't reach a statistical significance (q>0.1). TP53 was the top gene enriched in PD (10 out of 24 samples, p#=0.012 and p*=0.09) but didn't reach a statistical significance (q=0.23) along together with ATM, ARID1A, AHR, SMARCB1 (p #< 0.05). Three of nine RHOB mutations were in R (q=0.15, p=0.005 and p=0.16) and 2 out 6 ARID1A (p #< 0.05).

When the same random-permutation method was applied for the association of gene mutations to overall mutation burden (Mann-Whitney p-value was used as a statistic for each permutation) only ERCC2 was near significant (q=0.11, p#=0.003 and p*=0.00014), suggesting that the higher mutation burden in GO (vs PD/R) was partially explained with enriched ERCC2 mutations. In addition, ERBB3, KMT2C, and TP53 were also enriched in high mutation burden samples with p #< 0.05 although nominal p-values by Mann-Whitney were quite significant (Fig. 2).

## 4. Alterations in DNA Damage Response Genes

Four out of the 17 SMGs were genes involved in the DNA-damage response (DDR) and genomic regulation (ERCC2 16%, BRCA2 11%, STAG2 13%, ATM 10%).To better understand the role of DNA damage and repair processes in HGT1 there were assessed 34 genes encompassing major DNA repair pathways (table 2).

Table 2: DDR Gene Panel

| MMR | NER | HR | FA | Checkpoint | Others |
|-----|-----|-----|-----|-----|-----|
| MLH1 | ERCC2 | BRCA1 | BRCA2 | ATM | POLE |
| MSH2 | ERCC3 | MRE11A | BRIP1 | ATR | MUTYH |
| MSH6 | ERCC4 | NBN | FANCA | CHEK1 | PARP1 |
| PMS1 | ERCC5 | RAD50 | FANCC | CHEK2 | RECQL4 |
| PMS2 | | RAD51 | PALB2 | MDC1 | |
| | | RAD51B | RAD51C | | |
| | | RAD51D | BLM | | |
| | | RAD52 | | | |
| | | RAD54L | | | |

MMR: Mismatch repair, NER: Nucleotide excision repair, HR: Homologous recombination, FA: Fanconi anemia; MLH1: mutL homolog 1, Gene ID: 4292; MSH2: mutS homolog 2, Gene ID: 4436; MSH6:mutS homolog 6, Gene ID: 2956; PMS1: PMS1 homolog 1, mismatch repair system component, Gene ID: 5378; PMS2: PMS1 homolog 2, mismatch repair system component, Gene ID: 5395; ERCC3: ERCC excision repair 3, TFIIH core complex helicase subunit, Gene ID: 2071; ERCC4: ERCC excision repair 4, endonuclease catalytic subunit, Gene ID: 2072; ERCC5: ERCC excision repair 5, endonuclease, Gene ID: 2073; BRCA1: BRCA1 DNA repair associated, Gene ID: 672; MRE11A: MRE11 homolog, double strand break repair nuclease, Gene ID: 4361; NBN: nibrin, Gene ID: 4683; RAD50: RAD50 double strand break repair protein, Gene ID: 10111; RAD51: RAD51 recombinase, Gene ID: 5888; RAD51B: RAD51 paralog B, Gene ID: 5890; RAD51D: RAD51 paralog D, Gene ID: 5892; RAD52: RAD52 homolog, DNA repair protein, Gene ID: 5893; RAD54L: RAD54 like, Gene ID: 8438; BRCA2: BRCA2 DNA repair associated, Gene ID: 675; BRIP1: BRCA1 interacting protein C-terminal helicase 1, Gene ID: 83990; FANCA: FA complementation group A, Gene ID: 2175; FANCC: FA complementation group C, Gene ID: 2176; PALB2: partner and localizer of BRCA2, Gene ID: 79728; RAD51C: RAD51 paralog C, Gene ID: 5889; BLM: BLM RecQ like helicase, Gene ID: 641; ATM: ATM serine/threonine kinase, Gene ID: 472; ATR: ATR serine/threonine kinase, Gene ID: 545; CHEK1: checkpoint kinase 1, Gene ID: 1111; CHEK2: checkpoint kinase 2, Gene ID: 11200; MDC1: mediator of DNA damage checkpoint 1, Gene ID: 9656; POLE: DNA polymerase epsilon, catalytic subunit, Gene ID: 5426; MUTYH: mutY DNA glycosylase, Gene ID: 4595; PARP1: poly(ADP-ribose) polymerase 1, Gene ID: 142; and RECQL4: RecQ like helicase 4, Gene ID: 9401. (The Gene ID correspond to the GenBank® database on the 21st May 2019).

It was found that 60% of samples (36 out of 62) had at least one non-silent DDR gene mutation and the presence of DDR gene mutations was associated with higher mutation burden (p < 0.0001 Mann-Whitney) (Fig. 3). ERCC2 missense mutations were the most common DDR gene alteration, occurring in 16 % (10 out of 62) of the cases. In addition, ERCC2-mutated tumors had a significantly higher mutational burden than ERCC2 wild-type tumors and were seen at higher frequency in the GO (21/32, 65%) group compared to R (4/10, 40%) and PD (10/18, 55%) (p=0.0009), with similar pattern to overall TMB. When focusing on NER genes (where ERCC2 belongs) it was found that mutations in this group (after correcting for mutational burden) to be predictors of good outcome.

## 5. Mutational Signature Analysis

Exploiting a Bayesian variant of the non-negative matrix factorization (NMF) algorithm (SignatureAnalyzer) it was performed a *de novo* mutational signature analysis of the 62 samples for 27,477 SNVs stratified by 96 base substitution types, identifying five distinct mutational signatures

Five distinct mutational signatures were identified (COSMIC 1, 2, 5, 10 and 13): two signatures resembling those attributed to APOBEC activity (COSMIC2 + COSMIC13, APOBEC-a and APOBEC-b, respectively), a signature attributable to POLE signature (COSMIC10), a signature representing the superposition of the C>T CpG signature (COSMIC1), and a signature that resembled COSMIC signature 5. APOBEC-a and APOBEC-b accounted for 30% and 16% of all mutations respectively. POLE signature accounted for 16%; COSMIC1 signature with a predominance of C>T_CpG (5%), and COSMIC5 signature accounted for 34%. APOBEC-mediated mutagenesis was one of the major mutagenic sources in this cohort.

The activity of COSMIC5 signature was significantly higher in ERCC2 mutant samples of the cohort (median 280 vs 87, p=0.0002 by one-tailed Mann-Whitney). The statistical significance remained persistent when the analysis was repeated with a correction for overall mutation burdens across samples (q < 0.1 and p#=0.002 by random-permutation test) together with KMT2C. Also, ERCC2 mutation was associated with overall somatic single nucleotide variant mutations (SNVs) (p=#0.004 q=0.11 by random-permutation test), indicating that the increase of mutation burdens in GO was partly attributed to the activity of COSMIC5 (median 109 vs 85, p=0.22 by Mann-Whitney) in addition to the APOBEC mutagenesis. Notably the activity of COSMIC2 characterized by predominant C>T mutations at TCW (W=A/T) was significantly associated with GO (median 51 vs 16 between GO vs others, p=0.047 by Mann-Whitney) although the overall activity of APOBEC mutagenesis (COSMIC2 + COSMIC13) didn't reach a statistical significance (median 133 vs 89, p=0.21 by Mann-Whitney). Though GO samples presented high APOBEC all and COSMIC 5 activities, statistical significance was not reached either (p=0.09 and p=0.08, respectively). COSMIC 5 signatures in ERCC2 mutants was significantly associated with outcome (p=0.0002) compared to ERCC2 wild-type tumors. CT_CpG signature was higher in R, but overall contribution across samples was small. All signature enrichment analyses were performed without a single POLE mutated sample.

## 6. Mutational Signature Clustering Analysis

It was performed an unsupervised clustering analysis (as explained in the Material and Method section) based upon the activity of five mutational signatures and identified five mutational signature clusters, MSig1-5. One POLE sample with good outcome formed its own cluster (MSig5) due to the dominance of POLE signature. MSig1 formed the largest cluster (12 GO, 8 R, and 9 PD) and had the lowest median TMB (median 172 vs 448 between MSig1 vs others, $p<10^{-8}$ by Mann-Whitney). It had the lowest APOBEC mutagenesis activity (42 vs 239, $p<10^{-11}$ by Mann-Whitney) and the highest proportion of COSMIC1 (17 vs 7). Two putative MSI samples belonged to this cluster due to predominant C>T mutations at CpG resembling COSMIC1. MSig1 was associated the disease recurrence (p=0.04 by Fisher's exact test).

MSig2 formed the second largest cluster (n=18) with modest TMB (median 348, p=0.007 by Mann-Whitney) and intermediate APOBEC activity of median 234. This cluster presented a trend of association with good outcome (11 GO, 1 R, 6 PD) compared to MSig1.

All ERCC2 mutant samples (n=10) belonged to MSig3 (5 out of 7) and MSig2 (4 out of 19) (and the POLE sample). MSig3 presented the highest COSMIC5 signature activity (median 449 vs 87, p=0.00002 by Mann-Whitney), which could be responsible for the second highest TMB of median 647 after MSig4, and contained 5 ERCC2 mutant samples (p=0.0007 by Fisher's exact test). Interestingly, this cluster was associated with GO (all 7 samples belong to GO set, p=0.13).

MSig4 was characterized by very high TMB (median 777 vs 266, p=0.001 by Mann-Whitney) mainly attributed to the excessive APOBEC activity (median 646 vs 97, p=0.0002 by Mann-Whitney). Interestingly all samples in this cluster harbored TP53 mutations (8 missense, one splice-site, and one silent) and the likelihood of association of these mutations to two APOBEC signatures was significantly higher compared to TP53 mutations (20 missense, one nonsense, and one splice-site) in other MSig clusters (median 0.96 vs 0.17, p < 0.0001 by Mann-Whitney), suggesting that the excess of APOBEC mutagenesis was more likely responsible for these TP53 mutations. Despite only 6 samples in this cluster, they seemed to correspond to an aggressive cluster considering the relative proportion of poor outcomes with half of the patients progressing (3 PD, 1 R, and 2 GO), and the highest proportion of patients with pT1b (5 out of 6).

## 7. Somatic copy number alterations

Exome-wide copy-ratios were inferred using CAPSEG for 54 samples, and focal and arm-level somatic copy-number alterations (SCNAs) were further interrogated using GISTIC 2.0 (as explained in Material and Methods section). GISTIC analysis combined with manual inspection identified focal amplifications (log2(copy ratio) > 1) at PVRL4 (44%), YWHAZ (35%), CCND1 (26%), ERBB2 (22%), PPARG (22%), MDM2 (19%), SOX4 (19%), CCNE1 (17%), E2F3 (15%), AHR (13%), FGFR3 (6%), and EGFR (4%), and focal deletions (log2(copy ratio) < -0.7) at CDKN2A (31%), TSC1 (19%), and CREBBP (9%), and RB1 (6%). PVRL4 amplifications were the most frequent SCNAs (overall 44%) and relatively enriched in the recurrent tumors (70% in R vs 40% in PD or GO, p = 0.08 by one-tailed Fisher's exact test). Amplifications at 6p22.3 encompassing SOX4 and E2F3 (overall 15%) were more frequent in the progressor (33% in PD vs 13% in GO or R, p = 0.1 by one-tailed Fisher's exact test), while PPARG amplifications were more frequent in GO (29% in GO vs 12% in PD or R; p = 0.12 by one-tailed Fisher's exact test). CDKN2A deletions (overall 31%) were more frequent in PD or R tumors (44% in PD or R vs 18% in GO; p = 0.04 by Fisher's exact test) together with CCNE1 amplifications (28% in PD or R vs 7% in GO; p = 0.005 by Fisher's exact test), while three RB1 deletions were only present in GO.

An unsupervised hierarchical clustering of focal SCNA identified three main clusters. Cluster one was characterized by consistent loss of chromosome 9 and similar to the genomic subtype 2 (GS2) seen in Ta (Hurst CD, *et al.,* 2017). Cluster two was characterized by high frequency of CN events with a trend to find aggregated PD patients. A third cluster in between looked copy number quiet with no or few copy-number alteration as seen in GS1 of Ta (Hurst CD, *et al.,* 2017).

Many major events in clustering at the arm-level somatic copy number alterations (SCNAs) involved genes known to be altered in bladder cancer, including 9pq deletions, 20pq amplification, 8p amplification and 10q deletions. No particular patters or enrichments in outcome were observed in arm level SCNAs

## 8. Mutation and Copy Number Clusters

The TCGA 2014 study (Cancer Genome Atlas Research Network 2014 Nature) identified three biologically distinct groups in 125 MIBC samples based on mutations in SMGs and focal somatic copy number alterations (SCNAs) via an unsupervised NMF clustering; focally amplified' group with enriched focal SCNAs in several genes as well as mutations in KMT2D; CDKN2A-defident/FGFR3-altered' group with enriched papillary histology; 'TP53/cell-cycle-altered' group with TP53 mutations in nearly all samples as well as frequent RB1 mutations and amplifications in E2F3 and CCNE1 (Network CGAR, 2014). A similar analysis based on the genomic profiling with global gene expression also identified two major genomic circuits in mixed cohort of NMIBC and MIBC (Kim J, *et al.* 2015); one circuit was characterized by FGFR3 alterations, CCND1 over-expression, 9q and CDKN2A deletions, and the other was defined by E2F3 amplification, RB1 deletions and 5p gain. It was performed a consensus NMF-based clustering for 72 genetic alterations, comprising mutations in 46 genes (>= 5% across samples), focal amplifications and deletions in 16 genes, 10 recurrent arm-level gain or loss and consistently identified three DNA-based mutation and copy-number clusters, MutCN1-3 (see Materials and Methods).

MutCN1 was characterized by TP53 mutations and focal SCNAs in CCNE1, PVRL4, YWHAZ, E2F3-SOX4, PPARG, mainly corresponding to the 'TP53/cell-cycle-altered' group in MIBC; MutCN2 was associated with mutations in RB1 and ERCC2, and the chromatin-modifying genes CREBBP, KMT2D, KMT2C, and EP300.

MutCN3 was associated with mutations and amplification in FGFR3, CDKN2A deletions and amplifications in CCND1 and MDM2, and arm-level SCNAs in 9pq/17pq/20pq, which was analogous to the 'CDKN2A-deficient FGFR3 mutated' group in MIBC.

In addition to characteristic genetic alterations, MutCN clustering was also associated with clinical outcome (p=0.04 by Fisher's exact test); MutCN1 was more prone to progression or recurrence with a much higher proportion of PD (n=7, 37%) and R (n=5, 26%) compared to GO (n=7, 37%), while MutCN2 had largely favorable clinical outcome with the highest proportion of GO (n=16, 73%) over PD (n=6, 27%). The outcome association in MutCN3 was intermediate such as PD (n=5, 25%), R (n=5, 25%), and GO (n=10, 50%).

Consistently, MutCN clusters were also significantly associated with microstaging between pT1a versus pT1b (p=0.05 by Fisher's exact test), which was also linked with outcome in another clinical cohort (Orsola A, *et al.* 2015); The progression-prone MutCN1 harbored the highest proportion of pT1b (17 out of 19; 89%), while MutCN2 had a depletion of pT1b (12 out of 22, 55%) and the proportion of pT1b in MutCN3 was intermediate (14 out of 20, 70 %).

## 9. Prognostic association with good outcome

A multivariate logistic regression model was done looking for predictive clinical and genomic risk factors related to good outcome. ERCC2 mutations, NER mutations, DDR gene mutations, mutational signature, SNV number and microstaging (T1a versus T1b) were included in the model.

In the univariate analysis, the presence of DDR mutations was significantly correlated with GO (p 0.007). ERCC2 mutations alone were also significant (p=0.037 but removing ERCC2 mutations from the analysis of DDR mutations, the group lost its significance (p=0.15) meaning that mutations on ERCC2 were the ones driving the good outcome. ERCC2 lost its significance when the analysis was combined with TMB. Amongst the different mutational signatures contributing to GO, COSMIC 5 was the only one with a trend to contribute (p=0.056).

In terms of recurrence and progression, microstaging was not found to be significant (p=0.15) in the univariate analysis. A trend was seen for CDKN2A loss (p=0.08) and copy focal copy number gain in CCNE1 (p=0.07).

In the multivariate model, COSMIC 5 (enriched with ERCC2 mutations) showed a trend for good outcome (0.06) followed by APOBEC-a (p=0.07) but not all APOBEC combined (p=0.6).

Table 3: Main genomic characteristics predicting outcome in HGT1 bladder cancer (p#= empirical p value from the random permutation method correcting for heterogeneous mutation burdens among different outcome groups)

| | Good Outcome | Recurrent | Progression | Microstaging pT1b (deeper level of invasion) |
|---|---|---|---|---|
| **Tumor mutational burden** | High | Low | Intermediate | |
| **Mutation frequency analysis** | ERCC2 (p#<0.003, q =0.1)<br><br>BRCA2 (p#<0.05) but q>0.1<br>Mutations in DDR genes (p=0.0009) | RHOB and ARID1A (q>0.1, p#<0.05) | TP53, ATM, ARID1A, AHR, SMARCB1 (q>0.1, p# < 0.05) | |
| **Mutational Signature Analysis** | COSMIC2 (C>T mutations at TCW) (p=0.047)<br>COSMIC 5 in ERCC2 mutants (p=0.0002) | | | |
| **MSig clustering** | MSig3 (associated with ERCC2 mutations and COSMIC5) (7/7 GO) (p=0.13) | MSig1 (p=0.04) | MSig4 with high APOBEC activity and TP53 mutations (3/6 PD) | MSig4 (highest APOBEC with TP53 mutations) (5/6 pT1b) |
| **Copy number alterations** | | Focal PVRL4 amplification (p=0.08)<br>CDKN2A deletion (PD&R, p=0.04)<br>Focal CCNE1 amplification (PD&R, p=0.04) | | |
| **MutCN clusters** | MutCN2 in 73% of GO (p=0.04) | MutCN1 cluster-enrich-(PD&R, p=0.04) | | MutCN1 cluster in 89% of pT1b samples (p=0.05) (TP53, E2F3.amp, CCNE1.amp, other focal copy number events) (p=0.05) |
| (p#= empirical p value from the random permutation method correcting for heterogeneous mutation burdens among different outcome groups); (amp.: amplification). | | | | |

The main genomic characteristics predicting outcome in HGT1 bladder cancer were the following: progressive increase on TMB, decrease in the frequency of CM genes, ERCC2 mutations, increase on APOBEC-mediated mutagenesis, increase in frequency of TP53 mutations, decrease frequency of FGFR3 mutations, and KDM6A were seen more commonly in in low grade NMIBC (KDM6A was associated with GO), and KMT2D in higher frequency in HG NMIBC than LG NMIBC.

## 10. Conclusions:

The finding that high tumor mutational burden (TMB), ERCC2 mutations, COSMIC 2/ERCC2 mutations and COSMIC 5 signature were significant predictors of GO provide new opportunities to prospectively select and stratify patients in trials where less aggressive management and more conservative treatment could be offered. Alternatively, patients harbouring HGT1 tumors with TP53 mutations, early cystectomy could be more proactively recommended.

One of the most important observation in this cohort was that among patients with HGT1, TMB might be driving different outcomes. Mutational burden was found to be increased in GO (p=0.021) compared to PD, with R patients having the lowest mutational burden. This higher mutation burden in GO could be partially explained by mutations in the DDR gene ERCC2 (mutations were seen at higher frequency in the GO compared to R and PD, p=0.0009 and significance remained after correcting for TMB, q=0.1) and also partly attributed to the activity of COSMIC5 in addition to the APOBEC mutagenesis.

Another important observation was that ERCC2 (q=0.1) in addition to BRCA2 (pval=0.05) were the main drivers of GO, recognizing ERCC2 as an important predictor of good outcome.

As all the patients received BCG, it can also be hypothesized that high TMB was a predictive of intravesical immunotherapy (BCG).

In the series, TP53 was found mutated in 40% of patients, higher than what it was described in general series of NMIBC (22%) and close to the frequency seen in MIBC. It was found TP53 mutations more frequently in progressors versus non-progressors that could be explained by the insufficient high grade cases in these series, though this association did not pass multiple testing correction (q>0.1, p# < 0.05), there was an enrichment of TP53 mutations in PD. Mutations in ATM, ARID1A, AHR, SMARCB1 were also found more frequently in progressors (q>0.1, p# < 0.05).

In this cohort, the frequency of FGFR3 mutations (13%, 8 patients), PIK3CA (13%, 8 patients), RB1 (18%, 11 patients) and STAG2 (13%, 8 patients) was lower than what would be expected in NMIBC and similar to the frequency seen in MIBC in TCGA.

The frequency seen in mutations in chromatin modifier genes in these series of HGT1 patients was of 41% (7/17 SMG), inferior to Ta tumors and closer to the frequency in MIBC. The overall frequency was 24% also with female predominance (60% vs 15%). Also, mutations in KMT2D/MLL2 were seen in 27% of HGT1 unmatched samples.

The frequency of Ras superfamily GTPase member RHOB gene mutations in the HGT1 patients (14.5%) was similar to that seen in Ta NMIBC compared to 5% seen in MIBC and interestingly associated to disease recurrence (p# 0.005). ARID1A (p#< 0.05) was also significantly enriched in R recurrent patients. Some SMGs were found at a frequency not described in TCGA MIBC as BRCA2 (11%), EGFR (8%), CTNNB1 (8%), AHR (8%) and BRWD1 (8%). Other mutations described in TCGA MIBC were not seen in this cohort as CDKN1A, SPTAN1 (spectrin alpha, non-erythrocytic 1, Gene ID: 6709) or FAT1 (FAT atypical cadherin 1, Gene ID: 2195).

Interestingly, genes involved in the DNA damage response regulation were found mutated at a higher frequency than expected. 60% of samples (36 out of 62) had at least one non-silent DDR gene mutation and were associated with higher mutation burden and with GO (p=0.0009).

Mutational signature analysis identified five distinct mutational signatures being the APOBEC-mediated mutagenesis the major mutagenic process in this cohort. APOBEC activity in the HGT1 (about 46%) was higher than in Ta tumors but inferior to TCGA MIBC (67%). This progressive increase of the overall APOBEC activity might suggests that APOBEC mutagenesis might drive disease progression in NMIBC from low to high grade and subsequent progression to muscle invasive disease. The overall activity of APOBEC mutagenesis (COSMIC2 + COSMIC13) was associated with high mutational burden and enrichment of GO but didn't reach a statistical significance. Only APOBEC-a (C->T @ TCW) signature (COSMIC 2 signature), highly prevalent in this cohort of HGT1 (30%) was associated with GO (p=0.047). Also, the activity of COSMIC5 signature (34%) was significantly higher in ERCC2 mutant samples of this cohort (p#=0.004 q=0.11) together with KMT2C and was associated with GO (p=0.0002).

In the analysis of MSig clustering there were also identified five mutational signature clusters that were associated with disease outcome. MSig1 formed the largest cluster and had the lowest median TMB (p<10-8), the lowest APOBEC mutagenesis activity (p<10-11) and the highest proportion of COSMIC1 (17 vs 7). MSig1 was associated with disease recurrence (p=0.04 by Fisher's exact test) consistent with the associations of TMB and outcome (see above). MSig3 cluster was found to be driven by ERCC2 mutations and with a strong enrichment of COSMIC5 signature. Consistently, this cluster was associated with good outcome, with all 7 samples of this cluster having GO. The rest of ERCC2 mutated samples belonged to MSig2 signature (intermediate TMB) with a relative good outcome. Interestingly the MSig4 type with high TMB was found to have a very high APOBEC activity and all samples presented TP53 mutations. This cluster was observed more frequently in patients with pT1b, (deeper invasion into the lamina propria) and a trend to poor outcome.

It was seen in 15% of the samples and were more frequent in the progressor CDKN2A deletions (overall 31%) were more frequent in PD or R tumors (44% in PD or R vs 18% in GO; p = 0.04 by Fisher's exact test) together with CCNE1 amplifications (28% in PD or R vs 7% in GO; p = 0.0.05 by Fisher's exact test), while three RB1 deletions were only present in GO.

In multivariate analysis, a trend was seen for CDKN2A loss (p=0.08) to predict recurrence and progression.

MutCN1 was mainly characterized by TP53 mutations and enriched with focal SCNAs in CCNE1/PVRL4/E2F3/PPARG corresponding to the 'TP53/cell-cycle-altered' group in MIBC and linked with poor outcome. Indeed, MutCN1 was more prone to progression or recurrence while MutCN2 had largely favorable clinical outcome and MutCN3 being intermediate (p=0.04). Consistently, pathological microstaging pT1b (deep level of invasion into the lamina propria) was associated with MutCN1 type) (p=0.011) translating a more aggressive pattern of genomic alterations in pT1b versus pT1a.

MutCN3 was characterized by a good prognosis, showing high expression of early cell-cycle genes.

In summary, this was the first, homogeneously followed long-term cohort of genomically characterized HGT1 tumors in which patients were all prospectively followed (median follow-up 7 years) and received BCG under a pre-established protocol. Global findings confirmed that genomic alterations found in HGT1 were more close to alterations found in MIBC than to those seen in low grade or low stage NMIBC.

The clinical implications of these findings were the potential applicability to decide which patients could be safely offered conservative management while allowing forgoing cystectomy. For example, patients with favorable clinic pathological characteristics, with high TMB and enrichment with ERCC2 or DDR mutations can be eligible for a more conservative treatment approach. COSMIC 2 signature had good prognostic role, ERCC2 mutations-COSMIC 5 signature emerged as predictive of good outcome. COSMIC 2 and COSMIC 5 signatures could also help on the decision.

Limitations on the sample size of patients with evaluable genomic data led to only to a trend with lack of significant association between microstaging and outcome (p=0.7). Interestingly, specific DNA mutation pattern/copy number cluster (MutCN1 cluster) was associated with deeper level of invasion in HGT1 tumors. This was the first time that a pathological substaging had a defined correlative genomic profile highlighting the value of genomic finding predicting specific pathologic patterns of aggressiveness.

In conclusion, these findings of the integrative analysis of mutations, mutational signatures and copy number alterations can improve prediction of good outcome or the risk of recurrence or progression in HGT1 NMIBC.

**Citation List**

Non Patent Literature

Martin-Doyle W, et al. 2015 J Clin Oncol 33(6):643-50.

Dyrskjøt L, et al. 2017 Eur Urol 72(3):461-9.

Alexandrov LB et al. 2013 Nature 500(7463):415-421.

Martin-Doyle W, et al. 2015 J Clin Oncol 33(6):643-50.

Li H, et al. 2009 Bioinformatics 25(14):1754-60.

Strasky N, et al. 2011 Science 333(6046): 1157-60.

Cibulskis K et al. 2013 Nature Biotechnology 31(3) doi:10.1038/nbt.2514.

Robinson JT, et al. 2011 Nat Biotechnol 29(1):24-6.

van der Heijden AG, et al. 2016 Eur J Cancer 64:127-36.

Hedegaard J, et al. 2016 Cell 30(1):27-42.

Giacomelli AO, et al. 2018 Nat Genet 50:1381-1387.

Taylor-Weiner A, et al. 2018 Nat Methods 15(7):531-4.

Forbes SA, et al. 2015 Nucleic Acids Res 43(Database issue):D805-11.

Lek M, et al. 2016 Nature 536(7616):285-91.

Garofalo A, et al. 2016 Genome Med 8(1):79.

Lawrence MS et al. 2013 Nature 499(7457):214-218.

Mermel CH, et al. 2011 Genome Biol 12(4):R41.

Meeks JJ, et al. 2017 Cancer Cell 32(5):550-1.

Kim J, et al. 2016 Nat Genet 48(6):600-6.

Robertson AG et al. 2017 Cell 171 (3):540-556.e25.

Dyrskjøt L, et al. 2012 Br J Cancer 107(8):1392-8.

Taylor-Weiner A, et al. 2018 Nat Methods 15(7):531-4.

Strona G, et al. 2014 Nat Commun 5:4114.

Yoav Benjamini and Yosef Hochberg 1995 Journal of the Royal Statistical Society. Series B (Methodological) 57(1):289-300.

Pietzak EJ, et al. 2017 Eur Urol 72(6):952-959.

Hurst CD, et al. 2017 Cancer Cell 32(5):701-15.e7.

Cancer Genome Atlas Research Network (Network CGAR) 2014 Nature 507(7492):315-22.

Kim J, et al. 2015 Clin Cancer Res. 21 (20):4514-24.

Orsola A, et al. 2015 Br J Cancer 112(3):468-74.

## Claims

1. An *in vitro* method for the prediction of the outcome in a subject suffering High grade T1 (HGT1) non-muscle invasive bladder cancer (NMIBC), the method comprising the step of identifying at least a somatic mutation in the ERCC2 gene and/or identifying the COSMIC 2 signature in an isolated sample from the subject.

2. The *in vitro* method for the prediction of the outcome according to claim 1 which further comprises the step of identifying the COSMIC 5 signature.

3. The *in vitro* method for the prediction of the outcome according to any one of claims 1 or 2 wherein at least a somatic mutation in the ERCC2 gene and the COSMIC 5 signature are identified in the isolated sample; or, alternatively, at least a somatic mutation in the ERCC2 gene, the COSMIC 2 signature and the COSMIC 5 signature are identified in the isolated sample.

4. The *in vitro* method for the prediction of the outcome according to any one of claims 1 to 3, wherein when at least one somatic mutation is identified in the ERCC2 gene; or, alternatively, the COSMIC 2 signature is identified; or, alternatively, at least a somatic mutation in the ERCC2 gene and the COSMIC 5 signature are identified; or, alternatively, the COSMIC 2 signature and the COSMIC 5 signature are identified; or, alternatively, at least one somatic mutation of the ERCC2 gene, the COSMIC 2 signature, and the COSMIC 5 signature are identified in the test sample, it is indicative of good outcome.

5. The *in vitro* method for the prediction of the outcome according to any one of claims 1 to 4 which further comprises one or more of the following steps, in any order:

a. Identifying at least one somatic mutation in one or more genes selected from the following list: DNA-damage response gene, RB1, CREBBP, KMT2D, KMT2C, EP300, TP53;

b. Determining the copy number of one or more genes selected from the following list: RB1, DNA-damage response gene, CCNE1, CDKN2A, PVRL4, YWHAZ, E2F3, SOX4 and PPARG;

c. Evaluating one or more clinical-pathologic factor selected form the following list: lamina propria invasion, lymphovascular invasion, associated carcinoma *in situ,* use of bacillus Calmette-Guerin (BCG), and tumor size.

**6.** The *in vitro* method for the prediction of the outcome of HGT1 NMIBC according to claim 5 wherein the prediction is of good outcome when:

- at least a somatic mutation is identified in ERCC2 and in the one or more DNA-damage response genes provided that when the DNA-damage response gene is a check-point gene it is selected from ATR, CHEK1, CHEK2 and MDC1; or, alternatively,
- at least a somatic mutation is identified in the ERCC2 and in at least a DNA-damage response gene which is a nucleotide excision repair gene; or, alternatively,

at least a somatic mutation in one or more of ERCC2, ERCC3, ERCC4 and ERCC5 genes is identified; or, alternatively,

at least a somatic mutation in ERCC2 and BRCA2 genes are identified; or, alternatively,

at least one somatic mutation in ERCC2, CREBBP, KMT2D, KMT2C, EP300 and in the RB gene, or, alternatively, at least one somatic mutation in ERCC2 and in KMT2C as well as the COSMIC 5 signature are identified.

**7.** An *in vitro* method for deciding or recommending a medical regime to a subject with HGT1 NMIBC, the method comprising the step of identifying at least one somatic mutation of the ERCC2 gene and/or the COSMIC 2 signature; or, alternatively, at least a somatic mutation of the ERCC2 gene and the COSMIC 5 signature; or, alternatively, at least a somatic mutation of the ERCC2 and at least one DNA-damage response gene somatic mutation, wherein when it is a check-point gene it is selected from ATR, CHEK1, CHEK2 or MDC1; or, alternatively, at least a somatic mutation of the ERCC2 and at least one DNA-damage response gene somatic mutation wherein it is a nucleotide excision repair gene somatic mutation; or, alternatively, at least a somatic mutation of the ERCC2, ERCC3, ERCC4 and ERCC5 genes; or, alternatively, at least a somatic mutation of ERCC2 and BRCA2 genes; or, alternatively, the COSMIC 2 signature and the COSMIC 5 signature; or, alternatively, the COSMIC 2 signature, at least one somatic mutation of the ERCC2 gene and the COSMIC 5 signature, or, alternatively, at least a somatic mutation in ERCC2, CREBBP, KMT2D, KMT2C, EP300 and in the RB1 gene; or, alternatively, at least one somatic mutation in ERCC2, KMT2C and the COSMIC 5 signature are identified; in an isolated biological sample of the patient; and (ii) when the somatic mutation(s) is(are) identified, the medical regime decided or recommended is transurethral resection of bladder tumor (TURBT) and/or intravesical bacillus Calmette-Guerin (BCG).

**8.** The *in vitro* method for deciding or recommending a medical regime according to claim 7, which further comprises one or more of the following steps in any order:

- identifying at least one somatic mutation of the TP53 gene;
- determining the copy number of one or more genes selected from the following list: CCNE1, CDKN2A, PVRL4, YWHAZ, E2F3, SOX4 and PPARG;
- evaluating one or more clinical-pathologic factor(s) selected form the following list: lamina propria invasion, lymphovascular invasion, associated carcinoma *in situ,* use of bacillus Calmette-Guerin (BCG), and tumor size;

wherein, when no somatic mutation(s) as defined in claim 7 is detected, but at least one somatic mutation is detected in TP53, copy number gain of CCNE1 and/or CDKN2A deletion are identified; or, alternatively, when at least one somatic mutation in TP53 gene and SCNAs in CCNE1, PVRL4, YWHAZ, E2F3, SOX4 and PPARG are identified; or, alternatively, when one or more of lamina propria invasion, lymphovascular invasion, associated carcinoma *in situ,* non-use of bacillus Calmette-Guerin (BCG), and/or tumor size >3 cm, is detected, the medical regime decided or recommended is radical cystectomy.

**9.** The method according to any one of the claims 1-8, wherein the somatic mutation of ERCC2 is a mutation in the coding sequence.

**10.** The *in vitro* method according to any one of claim 1 to 9, wherein the identification of the somatic mutation is performed in the DNA comprised in the isolated sample.

11. The *in vitro* method according to any one of claim 1 to 10, wherein the sample is an isolated bladder tissue sample or a biological fluid.

12. The use of somatic mutations of the ERCC2 gene and/or of the COSMIC 2 signature; as marker(s) of prediction of outcome of HGT1 NMIBC or of deciding or recommending a medical regimen to a patient with HGT1 NMIBC.

13. The use of the combined use of at least one somatic mutation of the ERCC2 gene and of COSMIC 5 signature; or, alternatively, the COSMIC 2 signature and the COSMIC 5 signature; or, alternatively, at least one somatic mutation of the ERCC2 gene, the COSMIC 2 signature, and the COSMIC 5 signature, as marker of prediction of outcome of HGT1 NMIBC or of deciding or recommending a medical regimen to a patient with HGT1 NMIBC.

14. Use of means for identifying the somatic mutations as defined in any one of the claims 1 to 13, in any of the *in vitro* methods as defined in any of the claims 1 to 13.

15. The use of means according to claim 14, wherein said means form part of a kit.

Fig. 1

GO (n = 32)

PD (n = 18)

R (n = 10)

ERCC2 (8/9)

BRCA2 (5/6)

ATM (3/5)

TP53 (10/24)

ARID1A (3/6)
AHR
SMARCB1

RHOB (3/9)

ARID1A (2/6)

−Log10(empirical p-value)

Percentage (%)

Fig.2

**ERCC2 Signature**

Fig. 3

Europäisches
Patentamt

European
Patent Office

Office européen
des brevets

# EUROPEAN SEARCH REPORT

Application Number

EP 19 38 2527

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JAEGIL KIM ET AL: "Somatic ERCC2 mutations are associated with a distinct genomic signature in urothelial tumors", NATURE GENETICS., vol. 48, no. 6, 1 June 2016 (2016-06-01), pages 600-606, XP055456404, NEW YORK, US ISSN: 1061-4036, DOI: 10.1038/ng.3557 * page 605; figure 4 * | 1-15 | INV. C12Q1/6886 |
| X | PIETZAK EUGENE J ET AL: "Next-generation Sequencing of Nonmuscle Invasive Bladder Cancer Reveals Potential Biomarkers and Rational Therapeutic Targets", EUROPEAN UROLOGY, vol. 72, no. 6, 31 December 2017 (2017-12-31), pages 952-959, XP085263853, ISSN: 0302-2838, DOI: 10.1016/J.EURURO.2017.05.032 * abstract; figures 1,2 * | 1-15 | |
| X | FANTINI DAMIANO ET AL: "Genomic classification and risk stratification of bladder cancer", WORLD JOURNAL OF UROLOGY, SPRINGER INTERNATIONAL, DE, vol. 37, no. 9, 12 November 2018 (2018-11-12), pages 1751-1757, XP036875448, ISSN: 0724-4983, DOI: 10.1007/S00345-018-2558-2 [retrieved on 2018-11-12] * page 1751 - page 1752; figure 2 * | 1,4,7, 12,14,15 | TECHNICAL FIELDS SEARCHED (IPC)<br><br>C12Q |

-/--

The present search report has been drawn up for all claims

| Place of search | Date of completion of the search | Examiner |
|---|---|---|
| The Hague | 11 November 2019 | Reuter, Uwe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

## EUROPEAN SEARCH REPORT

Application Number

EP 19 38 2527

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | HEDEGAARD JAKOB ET AL: "Comprehensive Transcriptional Analysis of Early-Stage Urothelial Carcinoma", CANCER CELL, CELL PRESS, US, vol. 30, no. 1, 16 June 2016 (2016-06-16), pages 27-42, XP029636911, ISSN: 1535-6108, DOI: 10.1016/J.CCELL.2016.05.004 * abstract * * pages 28,35,39 * | 1-15 | |
| A,D | ROBERTSON A GORDON ET AL: "Comprehensive Molecular Characterization of Muscle-Invasive Bladder Cancer", CELL, vol. 171, no. 3, 19 October 2017 (2017-10-19), page 540, XP085233873, ISSN: 0092-8674, DOI: 10.1016/J.CELL.2017.09.007 * abstract; figures 2,7 * | 1-15 | |
| A | KENTARO INAMURA: "Bladder Cancer: New Insights into Its Molecular Pathology", CANCERS, vol. 10, no. 4, 1 April 2018 (2018-04-01), page 100, XP055639543, DOI: 10.3390/cancers10040100 * page 4 - page 5 * | 1-15 | TECHNICAL FIELDS SEARCHED (IPC) |

-/--

The present search report has been drawn up for all claims

| Place of search | Date of completion of the search | Examiner |
|---|---|---|
| The Hague | 11 November 2019 | Reuter, Uwe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 19 38 2527

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LUDMIL B. ALEXANDROV ET AL: "Signatures of mutational processes in human cancer. Supplementary information.", NATURE, vol. 500, no. 7463, 1 August 2013 (2013-08-01), pages 415-421, XP055456803, London ISSN: 0028-0836, DOI: 10.1038/nature12477 * pages s31,s61 * | 1-15 | |

TECHNICAL FIELDS
SEARCHED       (IPC)

The present search report has been drawn up for all claims

| Place of search | Date of completion of the search | Examiner |
|---|---|---|
| The Hague | 11 November 2019 | Reuter, Uwe |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ROBERTSON AG et al.** *Cell,* 2017, vol. 171 (3), 540-556
- **LAWRENCE MS et al.** *Nature,* 2013, vol. 499 (7457), 214-218
- **ROBERTSON AG et al.** *Cell,* vol. 171 (3), 540-556
- Nature. *Cancer Genome Atlas Research Network,* 2014
- *Network CGAR,* 2014
- **MARTIN-DOYLE W et al.** *J Clin Oncol,* 2015, vol. 33 (6), 643-50
- **DYRSKJØT L et al.** *Eur Urol,* 2017, vol. 72 (3), 461-9
- **ALEXANDROV LB et al.** *Nature,* 2013, vol. 500 (7463), 415-421
- **LI H et al.** *Bioinformatics,* 2009, vol. 25 (14), 1754-60
- **STRASKY N et al.** *Science,* 2011, vol. 333 (6046), 1157-60
- **CIBULSKIS K et al.** *Nature Biotechnology,* 2013, vol. 31 (3
- **ROBINSON JT et al.** *Nat Biotechnol,* 2011, vol. 29 (1), 24-6
- **VAN DER HEIJDEN AG et al.** *Eur J Cancer,* 2016, vol. 64, 127-36
- **HEDEGAARD J et al.** *Cell,* 2016, vol. 30 (1), 27-42
- **GIACOMELLI AO et al.** *Nat Genet,* 2018, vol. 50, 1381-1387
- **TAYLOR-WEINER A et al.** *Nat Methods,* 2018, vol. 15 (7), 531-4
- **FORBES SA et al.** *Nucleic Acids Res,* 2015, vol. 43, D805-11
- **LEK M et al.** *Nature,* 2016, vol. 536 (7616), 285-91
- **GAROFALO A et al.** *Genome Med,* 2016, vol. 8 (1), 79
- **MERMEL CH et al.** *Genome Biol,* 2011, vol. 12 (4), R41
- **MEEKS JJ et al.** *Cancer Cell,* 2017, vol. 32 (5), 550-1
- **KIM J et al.** *Nat Genet,* 2016, vol. 48 (6), 600-6
- **DYRSKJØT L et al.** *Br J Cancer,* 2012, vol. 107 (8), 1392-8
- **STRONA G et al.** *Nat Commun,* 2014, vol. 5, 4114
- **YOAV BENJAMINI ; YOSEF HOCHBERG.** Series B (Methodological). *Journal of the Royal Statistical Society,* 1995, vol. 57 (1), 289-300
- **PIETZAK EJ et al.** *Eur Urol,* 2017, vol. 72 (6), 952-959
- **HURST CD et al.** *Cancer Cell,* 2017, vol. 32 (5), 701-15
- Nature. *Cancer Genome Atlas Research Network (Network CGAR),* 2014, vol. 507 (7492), 315-22
- **KIM J et al.** *Clin Cancer Res.,* 2015, vol. 21 (20), 4514-24
- **ORSOLA A et al.** *Br J Cancer,* 2015, vol. 112 (3), 468-74